(19) Europäisches Patentamt European Patent Office Office européen des brevets

(11) **EP 3 214 443 A1**

(12) **EUROPEAN PATENT APPLICATION**
published in accordance with Art. 153(4) EPC

(43) Date of publication:
06.09.2017 Bulletin 2017/36

(51) Int Cl.:
*G01N 33/564* (2006.01)    *C07K 14/42* (2006.01)
*C12N 9/64* (2006.01)      *G01N 33/53* (2006.01)
*G01N 33/573* (2006.01)

(21) Application number: 15854205.0

(22) Date of filing: 29.10.2015

(86) International application number:
**PCT/JP2015/080522**

(87) International publication number:
**WO 2016/068226 (06.05.2016 Gazette 2016/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: 30.10.2014 JP 2014222096

(71) Applicants:
• **National Institute of Advanced Industrial Science and Technology**
Tokyo 100-8921 (JP)
• **Keio University**
Tokyo 108-8345 (JP)
• **GlycoBiomarker Leading Innovation Co., Ltd.**
Tsukuba-shi
Ibaraki 305-8568 (JP)

(72) Inventors:
• **KUNO, Atsushi**
Tsukuba-shi
Ibaraki 305-8568 (JP)
• **MATSUDA, Atsushi**
Tsukuba-shi
Ibaraki 305-8568 (JP)
• **NARIMATSU, Hisashi**
Tsukuba-shi
Ibaraki
3058568 (JP)
• **TAKEUCHI, Tsutomu**
Tokyo 160-8582 (JP)
• **SUZUKI, Katsuya**
Tokyo 160-8582 (JP)
• **TAKESHITA, Masaru**
Tokyo 160-8582 (JP)

(74) Representative: **Ladendorf, Oliver**
**Kraus & Weisert**
**Patentanwälte PartGmbB**
**Thomas-Wimmer-Ring 15**
**80539 München (DE)**

(54) **RHEUMATOID ARTHRITIS MARKER**

(57)    The present invention addresses the problem of providing a method whereby disease activity of rheumatoid arthritis is evaluated reliably and with good sensitivity by a simple method, and a kit for use in the method. The present invention furthermore provides a method for accurate diagnosis of polymyalgia rheumatica and relapsing polychondritis, which are similar rheumatic diseases, a method for differentiating rheumatoid arthritis, and a kit for use in the same. The present invention provides a Jacalin-binding O-linked oligosaccharide epitope as a negative marker for diagnosing rheumatoid arthritis, and enables more accurate assessment of disease condition by more objectively determining disease activity of rheumatoid arthritis using the amount of variation of a value obtained by multiplying the amount of MMP-3 in the blood or the amount of bound MMP-3 and ABA, ACA, or ACG in the blood by the reciprocal of the amount of bound MMP-3 and Jacalin in the blood. Through the discovery that an LEL or STL-reactive sugar chain on MMP-3 in the blood is a sugar chain marker for diagnosing polymyalgia rheumatica and relapsing polychondritis, the present invention also provides a method for accurate diagnosis of polymyalgia rheumatica and relapsing polychondritis. The present invention furthermore provides a method for accurate diagnosis of rheumatoid arthritis capable of differentiating between similar rheumatic diseases, by measuring the ratio of the amount of LEL or STL bound on MMP-3 and the amount of Jacalin, ACG, or the like bound on MMP-3 in the blood.

EP 3 214 443 A1

**Description**

Technical Field

The present invention relates to a simple and accurate method for evaluating disease activity of rheumatoid arthritis, and a simple and accurate kit for evaluation of disease activity of rheumatoid arthritis.

Background Art

Rheumatoid arthritis (RA) is a disease with high frequency by which nearly 1% of the population is affected, and is a progressive and refractory inflammatory disease generating joint destruction along with the joint synovial proliferation. Synovitis occurs in the joints of the whole body, inflammatory cytokines such as IL-1$\beta$ and TNF-$\alpha$, and various proteases are secreted from proliferated synovial tissues, gradually articular cartilage and bones are affected, and as the affection progresses, the joints are destroyed and deformed. Extracellular matrix metalloproteinases (MMPs) secreted from the chondrocytes or synovial cells activated by inflammatory cytokines are strongly involved in cartilage destruction, among them, MMP-3 (matrix metalloproteinases-3) are produced in a large amount in synovial cells of rheumatoid arthritis, and are known to be strongly involved in the progress of cartilage destruction (Non-Patent Documents 1 to 3). Further, those inflammatory cytokines cause also bone destruction via activation of osteoclasts, and suppression of osteoblasts, and cause dysfunction due to the joint destruction, therefore, it is known that the activities of daily living (ADL) of a patient are remarkably lowered (Non-Patent Document 4).

For a patient with rheumatoid arthritis, a drug therapy with the use of in addition to a corticosteroid, a nonsteroidal anti-inflammatory drug (NSAID), a disease-modifying antirheumatic drug (DMARD), a biological agent (an anti-TNF receptor antagonist, an anti-IL-1$\beta$ antagonist, or an anti-IL-6 receptor antagonist) and the like, is performed. However, in any one of the drugs, the effect is largely different among the individuals, and there are some cases in which remission is not achieved even if multiple drugs are used.

In such a treatment of refractory rheumatoid arthritis, it is important to suppress the progress of joint destruction by restrain the disease at least low disease activity or lower with the early detection and the early treatment (Non-Patent Document 5).

However, clinical findings of rheumatoid arthritis are largely different among the individuals, there has been no specific test yet, and differentiation from other arthritis is also difficult, therefore, it is considered that specialized knowledge is required for the early diagnosis, and continuous medical consultation by a specialist is required, also in the determination of the therapeutic effect in a similar way (Non-Patent Document 6).

In order to quantify and evaluate the disease activity of rheumatoid arthritis, various evaluation methods have been developed from the past, but accurate quantification is difficult. Among them, DAS (Disease Activity Score) developed as a technique for accurately quantitatively evaluating the disease activity of rheumatoid arthritis is a method in which the tender joint count and the swollen joint count are examined, and taking the self-evaluation of the physical condition of a patient into account, the determination is performed by applying the results to special calculation formulas. DAS has become the mainstream as a method for evaluating disease activity in clinical research.

However, it is indispensable to consult a skilled doctor about 28 joints, and in addition to that, complicated calculation using square root and logarithms is required, therefore, a problem in which it takes time in routine practice, symptoms of ankle joints and toe joints are not reflected, and the like has been pointed out (Non-Patent Document 7). Even if it is evaluated as "remission" with "DAS28", there may be a case where j oint destruction progresses, therefore, it is required to apply separately to Boolean criteria, and criteria of SDAI and the like as the clinical remission criteria (Non-Patent Document 8).

Accordingly, it is desired to search for a rheumatoid arthritis marker that is simple, and capable of accurately evaluating rheumatoid arthritis without overlooking the serious disease activity of joint destruction, and to develop a new evaluation method using the rheumatoid arthritis marker, and the active research and development have been conducted.

For example, detection of myelin basic protein autoantibody, and myelin basic protein gene polymorphism (Patent Documents 1 and 2), detection of F1 domain or F3 domain of a novel polypeptide Talin (Patent Document 3), detection of leucine-rich $\alpha$2 glycoprotein (LRG) (Patent Document 4), detection of a novel protein Synoviolin that expresses highly in synovial cells and relates to abnormal proliferation of synovial cells, and the gene and the autoantibody of the Synoviolin (Patent Documents 5 to 8), detection of $\gamma$-glutamyl transpeptidase ($\gamma$-GTP) and the gene thereof (Patent Document 9), measurement of the content of selenocysteine-containing proteins (Patent Document 10), detection of an anti-aldolase A antibody (Patent Document 11), diagnosis of type IX collagen destruction by an antibody detecting the cleavage position of the cleaved protein fragment of a type IX collagen $\alpha$-1 chain (Patent Document 12), detection of an auto antibody by an inositol 1,4,5-trisphosphate receptor (IP$_3$R) (Patent Document 13), detection of a chronic rheumatoid arthritis patient-specific antibody by a clone A polypeptide that is a RA antigen, and a follistatin-relatedprotein (FRP) (Patent Document 14), and the like have been proposed, and further detection of miRNA (miR124a), expression of which RA synovial cell-specifically increases or decreases (Patent Document 15) has also been proposed. Further, a method for differentiating osteoarthritis and rheumatoid arthritis by an antibody detecting the selective splicing position and amount of tenascin C in joint fluid (Patent Document 16) has also been proposed. However, there is yet no disease activity evaluation systemmarker that hasbeenpracticallyused.

Among these rheumatoid arthritis markers, currently, an MMP-3 level in serum is attracting attention as the most reliable prognostic factor for joint destruction (Non-Patent Document 9), and is clinically also used for the early diagnosis of rheumatoid arthritis, the determination of therapeutic strategy, and the like. Serum CRP and serum ESR, which have been conventionally used as an inflammatory marker for diagnosis of rheumatoid arthritis, reflect systemic inflammation, in contrast, MMP-3 in serum is said to reflect the pathological condition of a local joint, and is exerted the effect of differentiating early RA from osteoarthritis, gout, traumatic arthropathy, and the like, which are difficult to be differentiated from early RA. On the other hand, the MMP-3 in serum shows a high level even in polymyalgia rheumatica (PMR) being a rheumatic disease as well, which is difficult to be differentiated from early RA, further shows a high level in various collagen diseases, kidney disease, and the like, therefore, cannot be said to have high specificity. Accordingly, in order to accurately diagnose rheumatoid arthritis, the MMP-3 in serum has to be used in combination with an inflammatory marker such as serum CRP (Non-Patent Document 10).

Research focusing on changes in glycan composition of a glycoprotein in serum is also active. Conventionally, there is a method in which a glycoprotein in serum, such as an immunoglobulin G glycoprotein is heat-denatured, and then the glycan is cut out, labeled and purified, and subjected to glycan analysis using high performance liquid chromatography by an ODS-silica column, subsequently diagnosis of rheumatoid arthritis is performed based on the changes in glycan profile from the samples of healthy subjects (Patent Document 17), but preparation of samples for analysis is complicated, and further there is a problem that the sensitivity is low. In recent years, focusing on a N-linked glycan secreted in serum, a diagnostic method based on the analysis using MS/MS spectrum has been proposed, in which the abundance of multiple N-linked glycans in which significant increase in the expression level is observed in a patient with rheumatoid arthritis, in particular, the abundance of a glycan having a glycan profile of $(Hex)_3(HexNAc)_3(Deoxyhexose)_1(NeuAc)_3 + (Man)_3(GlcNAc)_2$, and the abundance ratio of the structural isomers having different binding sites of a fucose (Deoxyhexose) of the glycan are used as a diagnostic marker for rheumatoid arthritis (Patent Document 18). However, most of these N-linked glycans are present also in the blood of healthy subjects, the characteristic increase of $(Hex)_3(HexNAc)_3(Deoxyhexose)_1(NeuAc)_3 + (Man)_3(GlcNAc)_2$ is observed also in the deterioration in pathological conditions of viral hepatitis patients (for example, Nakagawa T et al. Journal of Biological Chemistry 281 (40) 29797-29806 (2006), and many others), therefore, there is a problem with clinical utility as a diagnostic marker for rheumatoid arthritis.

Many evaluation methods for accurately performing diagnosis and prognostic prediction of rheumatoid arthritis by combining multiple rheumatoid arthritis markers and applying to special calculation formulas to be scored have also been proposed.

For example, a method in which the presence of rheumatoid arthritis is evaluated based on the first marker (SLC16A4) gene expression level information, and the second marker (SLCO2B1, PTPRM, SHB, or ATP9A) gene expression level information (Patent Document 19) has been proposed.

Recently, Crescendo Bioscience, Inc. in the United States has developed a technique in which diagnosis and monitoring of rheumatoid arthritis are performed by measuring 12 biomarkers including IL-6, EGF, VEGFA, LEP, SAA1, VCAM1, CRP, MMP-1, MMP-3, TNFRSF1A, RETN, and CHI3L1 in serum, and by using the disease activity index (DAI) scored by special calculation formulas (Patent Document 20, and Non-Patent Document 11). It can be said that the technique has high correlation with "DAS28", and enables more accurate diagnosis of rheumatoid arthritis without requiring any detailed diagnosis by a skilled doctor. However, a large amount of money is required for the measurement of as many as 12 biomarkers, and calculation by complicated calculation formulas is also required, therefore, the technique cannot be said to be a very practical technique in a clinical site.

Under these circumstances, it has been strongly desired to provide a novel rheumatoid arthritis biomarker, with which disease activity of rheumatoid arthritis can be accurately evaluated by simple measurement with as few biomarkers as possible without requiring any complicated calculation formulas.

In addition, particularly in the initial stage of onset, rheumatoid arthritis may exhibit symptoms similar to those in polymyalgia rheumatica (PMR) that is one of the rheumatic diseases (Non-Patent Document 12), and there are some cases in which discrimination between both is difficult. Accordingly, a technique for accurately discriminating between the rheumatoid arthritis and the polymyalgia rheumatica has been required.

On the other hand, among rheumatic diseases, relapsing polychondritis (RP) is an extremely rare disease, the symptom is characteristic, and it is known that there are active periods and inactive periods, in which the symptoms appear and disappear. Further, as a specific blood test method, there is a method of using an anti-collagen antibody, but the positive rate is not so high, and there is also a problem that positive appears even in rheumatoid arthritis (Non-Patent Document 13). Accordingly, it has been desired to provide an excellent marker and an excellent test method, with which accurate measurement can be performed regardless in the active period, or the inactive period.

Citation List

Patent Documents

Patent Document 1: WO 2012/144512
Patent Document 2: JP 2014-170012 A
Patent Document 3: JP 2011-157301 A
Patent Document 4: JP 2010-286279 A
Patent Document 5: JP 2007-325595 A
Patent Document 6: JP 2007-82545 A
Patent Document 7: JP 2007-75106 A
Patent Document 8: WO 02/52007
Patent Document 9: JP 2003-24099 A
Patent Document 10: JP 2003-26598 A
Patent Document 11: JP 11-94836 A
Patent Document 12: WO 2008/108723
Patent Document 13: WO 2008/001944
Patent Document 14: WO 97/17441
Patent Document 15: WO 2010/001632
Patent Document 16: JP 2004-138489 A
Patent Document 17: JP 8-228795 A
Patent Document 18: WO 2009/096403
Patent Document 19: JP 2012-152198 A
Patent Document 20: WO 2011/047358

Non-Patent Documents

Non-Patent Document 1: Lab. Invest. 1992 Jun; 66(6): 680-690
Non-Patent Document 2: J. Biol. Chem. 1986 Oct 25; 261(30): 14245-14255
Non-Patent Document 3: Inflammation and Regeneration 2004 May; 24 (3) : 154-160
Non-Patent Document 4: J. Immunol. Res. 2014; 2014: 263625
Non-Patent Document 5: Ann. Rheum. Dis. 2010 Apr; 69 (4) : 631-637
Non-Patent Document 6: Ann. Rheum. Dis. 2014 Mar; 73 (3) : 492-509
Non-Patent Document 7: Arthritis Rheum. 2012 May; 64(5): 1316-1322
Non-Patent Document 8: Arthritis Rheum. 2011 Mar; 63 (3) : 573-586
Non-Patent Document 9: J. Rheumatol. 2000 Dec; 27 (12) : 2761-2768
Non-Patent Document 10: Ann. Rheum. Dis. 2002 Feb; 61 (2) : 161-166
Non-Patent Document 11: Arthritis Care Res. 2012; 64: 1794-1803
Non-Patent Document 12: J. Autoimmun. 2014; Feb-Mar; 48-49: 76-8.
Non-Patent Document 13: Arthritis Rheum. 1990 Oct 33(19):1493-1500
Non-Patent Document 14: Mol. Cell. Proteomics 2009; 8 (1) : 99-108
Non-Patent Document 15: Biochemistry 1990 Nov 6; 29(44): 10261-10270
Non-Patent Document 16: Nature Methods 2005; 2: 851-856
Non-Patent Document 17: Clin. Chem. 2011 Jan; 57(1): 48-56

Summary of the Invention

Problems to be solved by the Invention

An object of the present invention is to provide a method for evaluating disease activity of rheumatoid arthritis with a simple method, highly sensitively, and reliably; and a kit for the method. Specifically, an obj ect of the present invention is to provide a method for evaluating disease activity of rheumatoid arthritis, a method for determining effects of rheumatoid arthritis treatment, and a method for determining remission of rheumatoid arthritis, with clinically acceptable performance in terms of applicability, sensitivity, and accuracy, by a simple technique capable of being applied in medical practice by using a novel rheumatoid arthritis marker; and a kit for these methods.

In addition, an object of the present invention is also to provide a method for determining accurate discrimination between rheumatoid arthritis and polymyalgia rheumatica (PMR) that is a rheumatic disease similar to the rheumatoid arthritis, and a kit for the method, and further a method for determining accurately relapsing polychondritis (RP) among the rheumatic diseases, and also a kit for the method.

Mean for solving the Problems

To solve the problems described above, in the intensive studies about a rheumatoid arthritis marker for a method for evaluating disease activity of rheumatoid arthritis, which can be applied in a clinical site, the present inventors have focused on MMP-3 in serum, which is considered to most reflect the pathological condition of local joints among various known markers, and detailed studies have been further added. As a result of which, the present inventors have found that there is a tendency that the secretion amount of MMP-3 in serum is increased in affection of rheumatoid arthritis, and along with this, the profile of the glycan is also changed largely.

The present inventors have experienced previously that there is a case where the profile of the glycan in a specific glycoprotein in serum is changed depending on the pathological conditions in affection of rheumatoid arthritis, and therefore have come up with a possibility that a change in the profile of the glycan of MMP-3 in serum appears depending on the disease activity of rheumatoid arthritis. However, the MMP-3 proteins in serum increase by the affection of rheumatoid arthritis, but are extremely small as around 100 ng/ml to 1000 μg/ml at most, and the abundance ratio for the total amount of glycoproteins in blood is extremely small as 1/100 to 1/1,000,000 or less. Therefore, in specific detection of glycan change of the glycan of MMP-3 from the glycan of all glycoproteins contained in serum, it is difficult to perform accurate measurement because the S/N ratio becomes low, and the sensitivity is low. Accordingly, the present inventors have constructed a method in which structure information of glycan of MMP-3 in a minute amount in serum is obtained by utilizing an antibody-overlay-lectin array method using an anti-MMP-3 antibody. When this technique is applied to a commercially available recombinant MMP-3 protein, even with around 100 pg to 1 ng of MMP-3 protein, it has been possible to obtain the minimum glycan structure information. This indicates that analysis with an acute sensitivity is possible even in a minute amount of MMP-3 (around 40 ng/ml) contained in the blood of a healthy subject, and the glycan change depending on the medical condition can be studied.

Next, analysis by an antibody-overlay-lectin array method is applied to the serum of a patient affected with rheumatoid arthritis, and the like, a lectin recognizing a glycan specific to a glycan of MMP-3 in serum of a patient is selected, and identification of a glycan that becomes a rheumatoid arthritis biomarker is tried.

As a result, in MMP-3 in serum of a patient with rheumatoid arthritis, significantly high signals are observed in ACG, Jacalin, ABA, and ACA of lectin. Herein, because lectins of Jacalin, ABA, and ACA other than ACG are apparently a lectin recognizing O-linked sugar chain (O-glycan), these results mean that in affection with rheumatoid arthritis, as a glycan of MMP-3 in serum, an O-linked glycan, that is, an O-linked glycan being recognized by a lectin of at least any one of the Jacalin, ABA, and ACA is present. Conventionally, as the glycan profile change in a serum glycoprotein, mainly a N-linked glycan has been attracting attention (Patent Document 18, and the like), and as for the glycan of MMP-3, there has been no report even on the presence of O-linked glycan, therefore, it is considered that the glycan has a novel glycan structure as a glycan structure of a glycan epitope specific to rheumatoid arthritis.

However, there may be a case that only with a glycan having a high expression level, glycans inherently possessed by MMP-3 are secreted regardless in the affection with rheumatoid arthritis, and along with the increase in a secretion level of MMP-3 into serum, an increase in signals is observed. Therefore, as to each lectin signal amount, when the correlation with the MMP-3 amount in serum is observed, all of ACG, ABA, and ACA have a high correlation with the expression level of MMP-3, but only the Jacalin lectin has a weak correlation, and a case where the Jacalin signal is remarkably low is observed in a group particularly with high expression level of MMP-3. When the correlation of the ABA and ACA that are highly correlated with MMP-3 with Jacalin is taken, the correlation between the ABA and the ACA is high, but the correlation of Jacalin with a lectin of either ABA or ACA is low.

These results may reflect the difference in glycan recognition between Jacalin and each of ABA and ACA. The present inventors thought from the findings so far that the difference in glycan modification to the 6-position of an N-acetylgalactosamine (GalNAc) residue bound to the peptide in the O-linked glycan has been reflected in the results. It has been found that particularly Jacalin cannot accept the modification to the 6-position of GalNAc of the O-linked glycan, and as a result of the modification, the reactivity to Jacalin is lost (Non-Patent Document 14). As the representative modification to the position, there is sialylation. Accordingly, by using some of the serum samples among the serums of the above-described patients, sialidase treatment is performed, the sialidase is a sialic acid-degrading enzyme. As a result of performing lectin array analysis to the samples before and after the sialidase treatment, in the sample after the sialidase treatment, Jacalin signals also show a correlation with the amount of MMP-3 in the similar manner as in ABA, and ACA.

From the above-described results, it was revealed that the blood MMP-3 level is increased, in affection with rheumatoid arthritis, but the sialylation to the GalNAc residue directly bound to the MMP-3 peptide in the O-linked glycan of the MMP-3 differs depending on a patient. Moreover, in the group of patients whose disease activity of rheumatoid arthritis has deteriorated, there is a remarkable tendency that the Jacalin signals weaken, therefore, the possibility that there is an inverse correlation between the deterioration of disease activity and the increase of sialylation.

Subsequently, the clinical usefulness of this difference is investigated, and as an index to capture the "qualitative change" of O-linked glycan of MMP-3, the ABA/Jacalin value is examined. As a result of statistically verifying the correlation between this value and the clinical parameters, there is a significant correlation between this value with all of the values of DAS28-CRP, DAS28-ESR, CDAI, SDAI, and doctor VAS (visual analogue scale), which are conventionally numerical values representing the disease activity of rheumatoid arthritis, and in particular, the correlation with DAS28 showed as high as $p \leq 0.0001$.

This means that according to an analysis technique of glycan of specific protein using a lectin as represented by a lectin array or a lectin-antibody sandwich ELISA method, only by measuring the reactivity of MMP-3 in serum to Jacalin as well as the reactivity to ABA, ACA or ACG and determining the ratio between the two, the disease activity of rheumatoid arthritis can be objectively grasped without requiring any diagnosis by a skilled doctor and without performing any complicated calculation by complicated calculation formulas, and the same diagnostic accuracy of rheumatoid arthritis as that of DAS28 can be provided.

In addition, the glycan recognized by Jacalin is O-linked glycan, and has a core structure-1 (Core 1: Gal($\beta$1,3)Gal-NAc) or a Tn antigen (GalNAc) of the O-linked glycan which is bound to a Ser/Thr residue in a peptide chain. Further, in a case where sialic acid is introduced at the 6-position of GalNAc present in the glycan, the reactivity to Jacalin is inhibited by the modification. On the other hand, ABA and ACA are also O-linked glycan recognition lectins as Jacalin, but the sialylation at the position can be recognized, or accepted (Kuno A et al Molecular& Cellular Proteomics 8 (1) 99-108 (2009)). That is, the above-described results are results strongly suggesting that in affection with rheumatoid arthritis, sialylation to the 6-position of GalNAc residue in a core structure-1 of the O-linked glycan possessed by MMP-3 in serum is increased.

In order to verify this point, by using a synovial fluid sample collected from a lesion joint of an actual patient with rheumatoid arthritis, when Jacalin signals according to the MMP-3 level are plotted, a profile showing a clean inverse correlation was shown. On the other hand, the synovial fluid sample obtained from a patient with osteoarthritis that is difficult to be discriminated from early rheumatoid arthritis showed the same profile as that of healthy subjects.

This result demonstrates that in affection with rheumatoid arthritis, along with the deterioration of the pathological conditions, Jacalin-binding glycan epitopes of MMP-3, which are produced in synovial cells in a synovial tissue, are decreased, that is, it demonstrates that the sialylation to the 6-position of GalNAc residue in a core structure-1 of the O-linked glycan of the MMP-3 is increased. At the same time, the phenomenon of decrease of Jacalin-binding O-linked glycan epitopes of MMP-3, which is observed in a serum sample of a patient affected with rheumatoid arthritis, is nothing other than the exact reflection of the changes in glycan structure of the MMP-3 derived from a synovial tissue. This demonstrates also that accurate diagnosis of rheumatoid arthritis can be performed with a serum sample that is less invasive for a subject.

The present inventors obtained the above-described findings, and thus have completed the present invention.

That is, according to the present invention, it can be said that a (negative) glycan biomarker of an inverse correlation with rheumatoid arthritis, which includes a Jacalin lectin-binding O-linked glycan epitope of MMP-3 in serum is provided. Specifically, by measuring a value obtained by correcting the reciprocal of Jacalin signals in blood (for example, serum) with the MMP-3 level or the ABA, ACA, or ACG signal level, the disease activity of rheumatoid arthritis can be evaluated. Alternatively, also by observing the degree of sialic acid (NeuNAc) modification to the 6-position of GalNAc residue of "Gal($\beta$1,3)GalNAc", which is a core structure-1 of a Jacalin-binding O-linked glycan of MMP-3 in blood (serum) (when the sialylation increases, the re activity to Jacalin decreases), the disease activity of rheumatoid arthritis can be evaluated.

In the method for evaluating disease activity of rheumatoid arthritis according to the present invention, the rheumatoid arthritis can be detected and discriminated with clinically acceptable performance in terms of applicability, sensitivity, and accuracy. Moreover, the disease activity of rheumatoid arthritis can be evaluated by a method capable of being applied in medical practice with a simple technique.

In the present invention, the decrease of rheumatoid arthritis glycan biomarkers in a test sample can be assayed by staining the labeled Jacalin, and further the labeled ABA, ACA and ACG, or by a sandwich method with the labeled MMP-3 or the labeled anti-MMP-3 antibody. As the anti-MMP-3 antibody used in the sandwich method, the commercially available ones (productname) can be used. In addition, in a case where the commercially available antibody is not used as the anti-MMP-3 antibody, based on the known sequence information of MMP-3, specific antibodies can be prepared by a known procedure. The antibody used in the present invention may be either a polyclonal antibody or a monoclonal antibody. These antibodies can be prepared in accordance with a conventional production method of antibodies or antiserum.

As the technique in order to analyze the glycan structure of Jacalin-binding O-linked glycan of MMP-3, which is a negative rheumatoid arthritis glycan biomarker of the present invention, glycan profiling by frontal affinity chromatography (FAC), lectin microarray, MS or $MS^n$ (mass spectrometry, or tandem mass spectrometry), or the like can be mentioned. Once the glycan structure is determined, based on the information, a positive rheumatoid arthritis glycan biomarker can be selected. The information on lectins can be available from lectin frontier database (LfDB), the website of Research Center for Medical Glycoscience, the National Institute of Advanced Industrial Science and Technology, or the like. Further, an antibody recognizing the glycan structure can also be used.

By using the method for evaluating disease activity of rheumatoid arthritis according the present invention, and by using a blood sample (serum or plasma) of a subject suspected to be suffering from rheumatoid arthritis, the evaluation of disease activity of rheumatoid arthritis can be performed with simple and high accuracy in medical practice. Further, as the least invasive sample, a blood sample is preferred, similar results are obtained also with a joint fluid (synovial fluid), and in addition, even with a body fluid derived from a subject, such as pleural effusion, ascites, lymph, saliva, or spinal fluid, it is natural that the diagnosis can be performed in the similar way. Furthermore, in the following explanation, blood sample is mainly described as a typical example, but the present invention is not limited to the blood samples.

The present invention includes a kit for evaluation of disease activity of rheumatoid arthritis, which is used for performing the method for evaluating disease activity of rheumatoid arthritis according the present invention, and is provided with an anti-MMP-3 antibody together with Jacalin, and a lectin selected from the group consisting of ABA, ACA, and ACG, wherein either the lectins or the antibody being labeled.

In the kit, a lectin array in which multiple lectins containing Jacalin, and any lectin selected from the group consisting of ABA, ACA, and ACG are immobilized on a substrate is preferably used, and each lectin is made into a biotinylated lectin, and a lectin array in a form immobilizing the biotinylated lectins on a streptavidin-coated substrate can also be prepared.

In addition, in relation to the polymyalgia rheumatica (PMR) and relapsing polychondritis (RP), which are rheumatic diseases as rheumatoid arthritis is, in order to accurately determine the presence or absence of affection with each of the two, and in order to examine a biomarker to perform accurate discrimination from rheumatoid arthritis, the results of lectin array profiling are reviewed. Lectins named LEL and STL which exhibited statistically significantly difference, and both recognize a polylactosamine structure are selected, and by using newly prepared serum samples derived from patients with rheumatoid arthritis (RA), polymyalgia rheumatica (PMR), and relapsing polychondritis (RP), the reactivity of an MMP-3 concentrated fraction with LEL and STL lectins is examined.

As a result, the following significant results were obtained, for both lectins, the reactivities of samples derived from patients with relapsing polychondritis (RP) are extremely high, next, the reactivities of samples derived from patients with polymyalgia rheumatica (PMR) are high, and there is almost no reactivity in samples derived from patients with rheumatoid arthritis (RA), and in samples derived from healthy control subjects (HC).

This indicates that the LEL or STL lectin-reactive glycan of MMP-3 becomes an excellent glycan biomarker for determination of affection with polymyalgia rheumatica (PMR) and/or relapsing polychondritis (RP).

Further, it was confirmed that by taking the ratio of LEL lectin signals to Jacalin or ACG signals, only the ratio from serum derived from a patient with rheumatoid arthritis (RA) shows a high numerical value, compared with those from serum derived from a patient with polymyalgia rheumatica (PMR), a patient with relapsing polychondritis (RP), and a healthy control subject (HC).

From the above-described results, by measuring the reactivity with the LEL or STL lectin-reactive glycan of MMP-3 in a serum sample, for example, with a sandwich assay of an anti-MMP-3 antibody and a LEL or STL lectin, polymyalgia rheumatica (PMR) and/or relapsing polychondritis (RP) are discriminated from rheumatoid arthritis (RA), and the presence or absence of the affection can be accurately diagnosed.

Further, by measuring the ratio of the reactive signals with a LEL or STL lectin-reactive glycan of MMP-3 in a serum sample, and the reactive signals with the O-linked glycan of MMP-3, such as Jacalin, and ACG, similar rheumatic diseases are discriminated, and the affection of rheumatoid arthritis (RA) can be accurately diagnosed.

The present inventors obtained the above-described findings, and thus have solved other problems of the present invention.

That is, the present invention includes the following.

[1] A method for determining disease activity of rheumatoid arthritis, including a process of measuring changes in a glycan structure of an O-linked glycan of MMP-3 in a body fluid sample.
The present invention can also be expressed as follows.
[1'] A method for providing data for determination of disease activity of rheumatoid arthritis, comprising: a process of measuring alteration of MMP-3 O-glycan structure in a body fluid sample.
[2] The method according to [1], wherein the measurement of alteration of MMP-3 O-glycan structure is performed by calculating the following glycosylation discrimination index, X or Y:

```
X = measured signal corresponding to the total amount of
MMP-3 in a body fluid sample / measured signal corresponding
to the total amount of Jacalin-binding MMP-3 O-linked glycan
in said body fluid sample,
```

or

```
Y = measured signal corresponding to the total amount of
Jacalin-binding O-linked MMP-3 glycan o in a body fluid sample
/ measured signal corresponding to the total amount of the MMP-3
in said body fluid sample,
```

the X and Y are obtained by multiplying the measured signal corresponding to the total amount of MMP-3 in a body fluid sample by the reciprocal of measured signal corresponding to the total amount of Jacalin-binding MMP-3 O-linked glycan in said body fluid sample, and by multiplying the reciprocal of measured signal corresponding to the total amount of MMP-3 in a body fluid sample by measured signal corresponding to the total amount of Jacalin-binding MMP-3 O-linked glycan in said body fluid sample, respectively.

[3] The method according to [2], wherein the determination is conducted as follows:

as a value of the glycosylation discrimination index X is higher, disease activity of rheumatoid arthritis is evaluated to be higher, or as a value of the glycosylation discrimination index Y is higher, disease activity of rheumatoid arthritis is evaluated to be lower.

[4] The method according to [2] or [3], wherein the measured signal corresponding to the total amount of Jacalin-bindingMMP-3 O-linked glycan is a value measured by using a Jacalin lectin.

[5] The method according to any one of [2] to [4], wherein the measured signal corresponding to the total amount of MMP-3 is expressed as a measurement value of the abundance of MMP-3 in the body liquid sample, or a measurement signal, wherein an intensity of measurement signal changes depending on the abundance of MMP-3 in the body fluid sample.

[6] The method according to [5], wherein the measured signal corresponding to the total amount of MMP-3 expressed as a measurement signal is a signal level measured by using an anti-MMP-3 antibody specifically binds to a polypeptide chain of MMP-3, or a signal level indicating an amount of MMP-3 in the body fluid sample bound to a lectin selected from the group consisting of ABA, ACA, and ACG.

[7] A method for determining disease activity of rheumatoid arthritis, including the following processes (1) to (5):

(1) measuring signals indicating an amount of MMP-3 in a body fluid sample bound to Jacalin;
(2) measuring signals indicating an abundance of MMP-3 in said body fluid sample, or an amount of the MMP-3 in said body fluid sample bound to a lectin selected from the group consisting of ABA, ACA, and ACG or an amount of the MMP-3 in said body fluid sample bound to an anti-MMP-3 antibody;
(3) calculating a glycosylation discrimination index X by multiplying the reciprocal of the measurement value obtained in the process (1) by the measurement value obtained in the process (2), or a glycosylation discrimination index Y by multiplying the measurement value obtained in the process (1) by the reciprocal of the measurement value obtained in the process (2) ;
(4) comparing the value (X) or (Y) calculated in the process (3) with a reference value ($X_0$) or ($Y_0$);
(5) determining to have high disease activity of rheumatoid arthritis or to be affected with rheumatoid arthritis, in a case of X being higher than the reference value $X_0$ in the process (4) ($X > X_0$), or

determining to have high disease activity of rheumatoid arthritis or to be affected with rheumatoid arthritis, in a case of Y being lower than the reference value $Y_0$ in the process (4) ($Y < Y_0$).

[8] The method according to [7], wherein the reference value $X_0$ or $Y_0$ is a value calculated for body fluid samples from healthy subjects through the processes of (1) to (3).

[9] A method for determining condition changes over time of disease activity of rheumatoid arthritis of a subject, including the following processes (1) to (5):

(1) measuring signals indicating an amount of MMP-3 in a body fluid sample bound to Jacalin;

(2) measuring signals indicating an abundance of MMP-3 in said body fluid sample, or an amount of the MMP-3 in said body fluid sample bound to a lectin selected from the group consisting of ABA, ACA, and ACG or an amount of the MMP-3 in said body fluid sample bound to an anti-MMP-3 antibody;

(3) calculating a glycosylationg discrimination index X' by multiplying the reciprocal of the measurement value obtained in the process (1) by the measurement value obtained in the process (2), or a glycosylationg discrimination index Y' by multiplying the measurement value obtained in the process (1) by the reciprocal of the measurement value obtained in the process (2);

(4) comparing the value (X') or (Y') calculated in the process (3) with a value $(X'_0)$ or $(Y'_0)$ calculated for a body fluid sample before the test day from the same subject through the processes of (1) to (3);

(5) determining disease activity of rheumatoid arthritis of the subj ect to be deteriorated, in a case of X' being a numerical value higher than the value before the test day $(X' > X'_0)$ or a case of Y' being a numerical value lower than the value before the test day $(Y' < Y'_0)$, and disease activity to be improved in a case of X' being a numerical value lower than the value before the test day $(X' < X'_0)$ or a case of Y' being a numerical value higher than the value before the test day $(Y' > Y'_0)$, in the comparison process of the process (4).

[10] The method according to [9], wherein the body fluid sample is a body fluid sample derived from a subject after rheumatoid arthritis treatment, and the body fluid sample before the test day is a body fluid sample derived from the same subject before rheumatoid arthritis treatment, and

in the comparison process of the process (4), the rheumatoid arthritis treatment applied to the subject is evaluated to have a therapeutic effect in a case of X' being lower than that before treatment $(X' < X'_0)$ or a case of Y' being higher than that before treatment $(Y' > Y'_0)$, and is evaluated not to have a therapeutic effect in a case of X' being higher than that before the treatment or no change $(X' \geq X'_0)$ or a case of Y' being lower than that before the treatment or no change $(Y' \leq Y'_0)$.

[11] A glycan epitope to be a biomarker for determination of disease activity of rheumatoid arthritis, wherein the glycan epitope is an MMP-3 O-linked glycan, and a Jacalin non-binding glycan.

[12] A glycan epitope to be a biomarker for determination of disease activity of rheumatoid arthritis, wherein the glycan epitope is an MMP-3 O-linked glycan , and a GalNAc structure of the glycan epitope bound to a Ser/Thr residue in an MMP-3 peptide chain is $\alpha2,6$-sialylated.

[13] A biomarker for determination of disease activity of rheumatoid arthritis, including the glycan epitope according to [11] or [12].

[14] An MMP-3 for use in a method for determining disease activity of rheumatoid arthritis, wherein the MMP-3 comprises multiple O-linked glycans in which GalNAc structure bound to a Ser/Thr residue in an MMP-3 peptide chain is $\alpha2,6$-sialylated.

[15] An MMP-3 for use in a method for determining disease activity of rheumatoid arthritis, wherein the MMP-3 comprises multiple Jacalin non-binding O-linked glycans and is fractionated from a body fluid sample.

[16] Use of an MMP-3 O-linked glycan for the method of assessing disease activity of rheumatoid arthritis, wherein a GalNAc structure of the MMP-3 O-linked glycan bound to a Ser/Thr residue in an MMP-3 peptide chain is $\alpha2,6$-sialylated.

[17] An MMP-3 O-linked glycan for use in a method of determining disease activity of rheumatoid arthritis, wherein a GalNAc structure of the MMP-3 O-linked glycan bound to a Ser/Thr residue in an MMP-3 peptide chain is $\alpha2,6$-sialylated.

[18] A reagent for assessing disease activity of rheumatoid arthritis capable of being applied to a body fluid sample which comprises the glycan epitope described in [11] or [12], or the substance specifically recognizing MMP-3 described in [14] or

[15]. [19] The reagent for assessing disease activity of rheumatoid arthritis according to [18], wherein the substance specifically recognizing the glycan epitope is a substance selected from the group consisting of a glycan-recognizing antibody, a lectin, a glycan-recognizing peptide, and a glycan-recognizing aptamer.

[20] A reagent for assessing disease activity of rheumatoid arthritis capable of being applied to a body fluid sample, including a substance which recognizes a glycan containing $\alpha$GalNAc structure bound to a Ser/Thr residue in an MMP-3 peptide chain, but does not recognize said glycan when $\alpha$GalNAc structure therein is $\alpha2,6$-sialylated.

[21] The reagent for assessing disease activity of rheumatoid arthritis according to [20], wherein the substance which recognizes a glycan containing $\alpha$GalNAc structure bound to a Ser/Thr residue in an MMP-3 peptide chain, but does not recognize said glycan when $\alpha$GalNAc structure therein is $\alpha2,6$-sialylated is Jacalin.

[22] A kit for assessing disease activity of rheumatoid arthritis, including the reagent for assessing disease activity of rheumatoid arthritis described in any one of [18] to [21], and further a substance which specifically binds to MMP-3 in a body fluid sample in an MMP-3 abundance-dependent manner.

[23] The kit for assessing disease activity of rheumatoid arthritis according to [22], wherein the substance which specifically binds to MMP-3 in a body fluid sample in an MMP-3 abundance-dependent manner is a lectin selected from the group consisting of ABA, ACA, and ACG, or an anti-MMP-3 antibody.

[24] Use of Jacalin in a method for assessing disease activity of rheumatoid arthritis.

[25] Jacalin for use in a method for assessing disease activity of rheumatoid arthritis.

[26] Use of Jacalin in production of a reagent for determination of disease activity of rheumatoid arthritis.

[27] A glycan epitope to be a biomarker for diagnosis of polymyalgia rheumatica (PMR) and/or relapsing polychondritis (RP), being a glycan of MMP-3 in a body fluid sample, and having binding activity to a lectin which recognizes a polylactosamine structure.

[28] The glycan epitope according to [27], wherein the lectin which recognizes a polylactosamine structure is a LEL lectin or a STL lectin.

[29] A biomarker for diagnosis of polymyalgia rheumatica (PMR) and/or relapsing polychondritis (RP) which comprises the glycan epitope according to [27] or [28].

[30] A reagent for diagnosing polymyalgia rheumatica (PMR) and/or relapsing polychondritis (RP) capable of being applied to a blood sample, comprising: a substance which specifically recognizes the glycan epitope according to [27] or [28], or MMP-3 containing said glycan epitopes.

[31] A method for diagnosing polymyalgia rheumatica (PMR) and/or relapsing polychondritis (RP), comprising: a process of measuring the abundance of glycan in a body fluid sample having binding activity against an anti-MMP-3 antibody as well as binding activity to a lectin which recognizes a polylactosamine structure, for a body fluid sample. The present invention can also be expressed as follows.

[31'] A method for providing data for diagnosis of polymyalgia rheumatica (PMR) and/or relapsing polychondritis (RP), comprising: a process of measuring the abundance of glycan in a body fluid sample having binding activity against an anti-MMP-3 antibody as well as binding activity to a lectin which recognizes a polylactosamine structure, for a body fluid sample.

[32] The method according to [31], wherein the lectin which recognizes a polylactosamine structure is a LEL lectin or a STL lectin.

Further, the present invention can also be expressed as follows.

[32'] Use of a LEL lectin or STL lectin in a method for diagnosing polymyalgia rheumatica (PMR) and/or relapsing polychondritis (RP).

[32"] A LEL lectin or STL lectin for use in a method for diagnosing polymyalgia rheumatica (PMR) and/or relapsing polychondritis (RP).

[32'''] A LEL lectin or STL lectin for use in a reagent for diagnosis of polymyalgia rheumatica (PMR) and/or relapsing polychondritis (RP).

[33] The method according to [31] or [32], wherein the measurement process is performed by a sandwich assay which employs an anti-MMP-3 antibody and a lectin recognizing a polylactosamine structure.

[34] A method for determining whether a subject suffers from polymyalgia rheumatica (PMR) and/or relapsing polychondritis (RP), characterized in that a body fluid sample derived from a subj ect suspected to be suffering frompolymyalgia rheumatica (PMR) and/or relapsing polychondritis (RP) is subjected to the following processes (1) to (3):

(1) measuring the abundance of glycan in a body fluid sample having binding activity against an anti-MMP-3 antibody as well as binding activity to a lectin which recognizes a polylactosamine structure;
(2) measuring the abundance of glycan in a body fluid sample derived from a healthy subject and/or a patient with rheumatoid arthritis having binding activity against an anti-MMP-3 antibody as well as binding activity to a lectin which recognizes a polylactosamine structure; and
(3) determining to be suffering from polymyalgia rheumatica (PMR) and/or relapsing polychondritis (RP) in a case where a numerical value showing the abundance of glycan obtained in (1) significantly exceeds a numerical value showing the abundance of glycan obtained in (2).

In addition, herein, a typical example of the lectin recognizing polylactosamine structure is a LEL lectin or STL lectin.

[35] The method according to [34] wherein the measurement processes of (1) and (2) are performed by a sandwich assay which employs an anti-MMP-3 antibody and a LEL lectin or STL lectin.

[36] A method for diagnosing rheumatoid arthritis (RA), comprising: applying to a body fluid sample at least the following measurement processes (1) and (2):

(1) measuring the abundance of glycan in a body fluid sample having binding activity against an anti-MMP-3 antibody as well as binding activity to a lectin which recognizes a polylactosamine structure; and
(2) measuring the abundance of glycan in said body fluid sample having binding activity against an anti-MMP-3 antibody as well as binding activity to at least one O-linked glycan-recognition lectin selected from the group consisting of Jacalin, ABA, ACA, and ACG.

In addition, herein, a typical example of the lectin recognizing polylactosamine structure is a LEL lectin or STL lectin. Furthermore, the present invention can also be expressed as follows.

[36'] A method for providing data for diagnosis of rheumatoid arthritis (RA), comprising: applying to a body fluid sample at least the following measurement processes (1) and (2) :

(1) measuring the abundance of glycan in a body fluid sample having binding activity against an anti-MMP-3 antibody as well as binding activity to a lectin which recognizes a polylactosamine structure; and
(2) measuring the abundance of glycan in said body fluid sample having binding activity against an anti-MMP-3 antibody as well as binding activity to at least one O-linked glycan-recognition lectin selected from the group consisting of Jacalin, ABA, ACA, and ACG.

[37] The method according to [36], wherein the measurement processes (1) and (2) are performed simultaneously by a sandwich assay which employs said an anti-MMP-3 antibody, and a lectin array containing a lectin which recognizes a polylactosamine structure and said O-linked glycan-recognition lectin.

[38] A method for determining whether a subject suffers from rheumatoid arthritis, characterized in that a body fluid sample derived from a subject suspected to be suffering from rheumatoid arthritis is subjected to the following processes (1) to (4):

(1) measuring the abundance of glycan in the body fluid sample having binding activity against an anti-MMP-3 antibody as well as binding activity to a lectin which recognizes a polylactosamine structure;
(2) measuring the abundance of glycan is said body fluid sample having binding activity against an anti-MMP-3 antibody as well as binding activity to at least one O-linked glycan-recognition lectin selected from the group consisting of Jacalin, ABA, ACA, and ACG;
(3) calculating a ratio of the abundance of glycan obtained in (2) to the abundance of glycan obtained in (1); and
(4) determining the subj ect to be suffering from rheumatoid arthritis in a case of that the numerical value of ratio obtained in the process (3) is significantly higher than a numerical value of ratio of healthy subjects which is obtained by applying the processes (1) to (3) to a body fluid sample derived from a healthy subject in advance or at the same time.

In addition, herein, a typical example of the lectin recognizing polylactosamine structure is a LEL lectin or STL lectin.

[39] The method according to [38], wherein the measurement processes (1) and (2) are performed simultaneously by a sandwich assay which employs said anti-MMP-3 antibody, and a lectin array containing a lectin which recognizes a polylactosamine structure and said O-linked glycan-recognition lectin.

[40] A kit for diagnosis of polymyalgia rheumatica (PMR) and/or relapsing polychondritis (RP), including the following (1) and (2) :

(1) an anti-MMP-3 antibody; and
(2) a lectin recognizing a polylactosamine structure.

[41] The kit according to [40], wherein the lectin recognizing a polylactosamine structure is a LEL lectin or a STL lectin.

[42] A kit for diagnosis of rheumatoid arthritis, including the following (1) to (3):

(1) an anti-MMP-3 antibody;
(2) a lectin recognizing a polylactosamine structure; and
(3) at least one lectin recognizing an O-linked glycan selected from the group consisting of Jacalin, ABA, ACA, and ACG.

[43] The kit according to [42], wherein lectins of (2) and (3) are present in the same lectin array.

Effects of the Invention

The method for evaluating disease activity of rheumatoid arthritis of the present invention, in which the decrease of Jacalin-binding O-linked glycan of MMP-3 is observed can objectively determine the disease activity of rheumatoid arthritis, and enables more accurate grasp of the medical condition. In addition, with a simple operation of only measuring the binding signals of Jacalin, and a lectin selected from the group consisting of ABA, ACA, and ACG, or an anti-MMP-3 antibody, in serum, it becomes possible that accurate determination equivalent to "DAS28" that requires detailed diagnosis by a skilled doctor and analysis by complicated calculation formulas, and the benefit in medical practice is immeasurable. Moreover, in the present invention, it was demonstrated that the qualitative changes of the O-linked glycan of MMP-3, which are observed in a body fluid sample such as a blood sample (serum sample), or a joint fluid (synovial fluid) sample accurately reflect the qualitative changes of the O-linked glycan of MMP-3 produced in a synovial tissue. According to the present invention, a kit for determination of disease activity of rheumatoid arthritis, which has simplicity and accuracy, is excellent in clinical application, and is capable of being easily applied in medical practice can be provided. With the kit, a possibility that early rheumatoid arthritis can be discriminated from other joint inflammatory diseases such as osteoarthritis, the discrimination has been conventionally difficult, is also expected.

In addition, according to the present invention, it has been found that a LEL or STL lectin-reactive glycan of MMP-3 in a body fluid sample becomes a glycan biomarker for accurate determination of the presence or absence of the affection with each of the polymyalgia rheumatica (PMR) and relapsing polychondritis (RP), which are rheumatic diseases as rheumatoid arthritis is, and by using a LEL lectin, and/or a STL lectin together with an anti-MMP-3 antibody, a method for accurately diagnosing polymyalgia rheumatica (PMR) and relapsing polychondritis (RP), and a method for discrimination of both of the two from rheumatoid arthritis have been provided.

Further, a method for accurately diagnosing rheumatoid arthritis (RA) and a method for discriminating rheumatic diseases similar to one another have been provided, including a process of measuring a ratio of a reactive signal indicating a LEL or STL lectin-reactive glycan of MMP-3 in a body fluid sample to a reactive signal indicating a lectin-reactive glycan of MMP-3 in a body fluid sample, wherein the lectin is an MMP-3 O-linked glycan recognition lectin selected from the group consisting of Jacalin, ACG, ABA, ACA, and ACG.

Furthermore, according to the present invention, by combining with an anti-MMP-3 antibody, and a lectin recognizing a polylactosamine structure such as a LEL or STL lectin, or further by combining with a lectin for the recognition of an O-linked glycan of MMP-3 selected from the group consisting of Jacalin, ACG, ABA, ACA, and ACG, a kit for diagnosis of polymyalgia rheumatica (PMR),relapsing polychondritis (RP),and rheumatoid arthritis (RA), which enables significant discrimination from rheumatic diseases similar to one another, can be provided.

Brief Description of Drawings

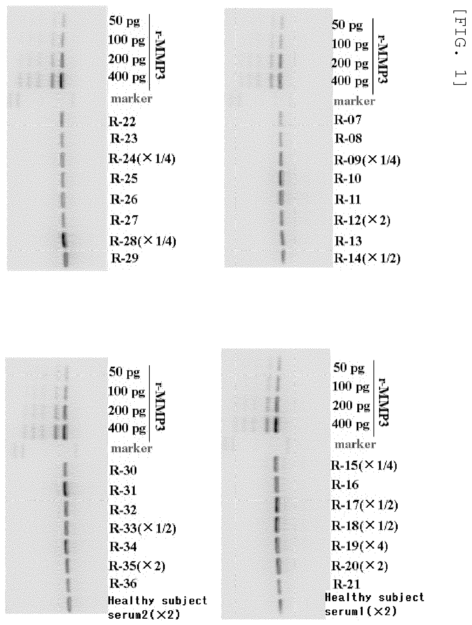

FIG. 1 shows Western blotting of eluted fractions (MMP-3-IP). In addition, the MMP-3 level in a sample is adjusted in the range of standard rMMP-3 (50 to 400 pg) by a pre-experiment in advance so as to be converted as a recombinant MMP-3 level, and then each serum sample is loaded into a gel. Inside the parentheses in the drawing shows an applied amount of the sample (non-indication: 0.1 $\mu$L (equivalent to 0.2 $\mu$L of serum), $\times$ 1/4: 0.025 $\mu$L (equivalent to 0.05 $\mu$L of serum), $\times$ 1/2: 0.05 $\mu$L (equivalent to 0.1 $\mu$L of serum), $\times$ 2: 0.2 $\mu$L (equivalent to 0.4 $\mu$L of serum), and $\times$ 4: 0.4 $\mu$L (equivalent to 0.8 $\mu$L of serum)).

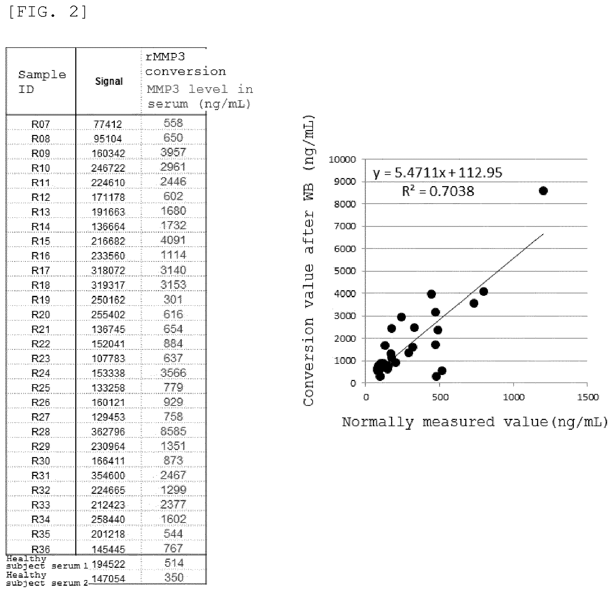

FIG. 2 shows a correlation diagram of an MMP-3 level estimated from the band intensities of Western blotting (WB) and a normally measured MMP-3 level.

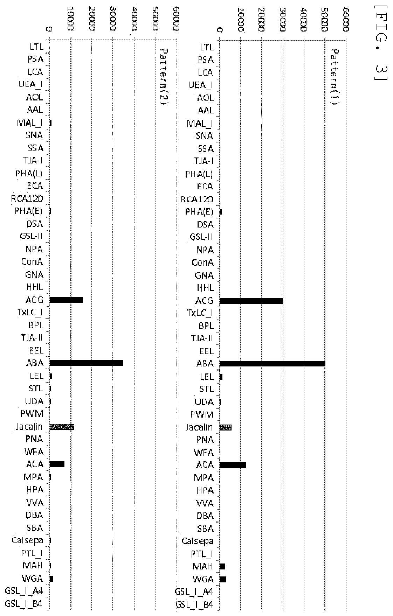

FIG. 3 shows results of lectin array analysis of the MMP-3 derived from serum of a patient, and graphs for two cases showing a typical profile among 36 cases measured. It can be seen that signal patterns of ACG that is an $\alpha$2,3 sialic acid recognizing lectin, and those of ABA and ACA that are O-linked glycan recognition lectins are extremely similar to each other, but in contrast, the relative intensity is different from that of Jacalin that is also an O-linked glycan recognition lectin. Among the cases, a case with a low Jacalin value is called "pattern (1)", and a case with a high Jacalin value is called "pattern (2)".

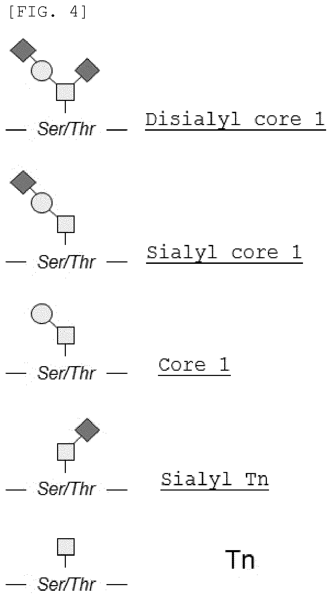

FIG. 4 shows major O-linked glycan structures of MMP-3 assumed from lectin array analysis. In the drawing, glycan structures underlined are assumed as the major O-linked glycan structures of MMP-3. In addition, □ represents GalNAc, ○ represents Gal, and ♦ represents NeuNAc.

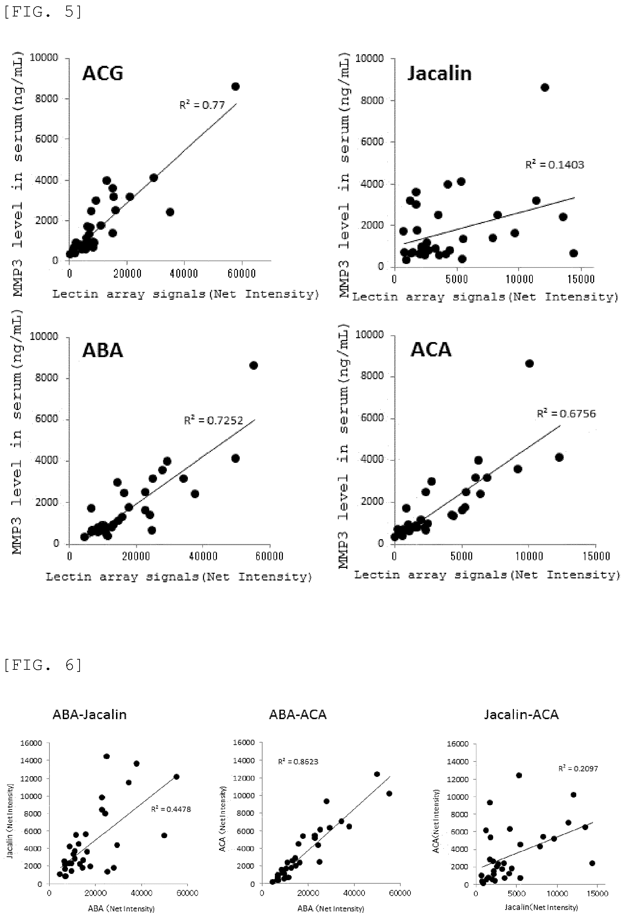

FIG. 5 shows correlation diagrams of the MMP-3 level in serum and the signal intensity of each of ABA, ACA, ACG, and Jacalin. The signals of any one of ABA, ACA, and ACG are correlated with the MMP-3 level, but no correlation was observed between the Jacalin signals and the MMP-3 level.

FIG. 6 shows correlation diagrams among lectin signals of ABA, ACA, and Jacalin. A strong correlation was observed between ABA and ACA, but no correlation was observed between Jacalin and any of the lectins, and it became clear that only Jacalin shows a tendency not correlated with the MMP-3 level.

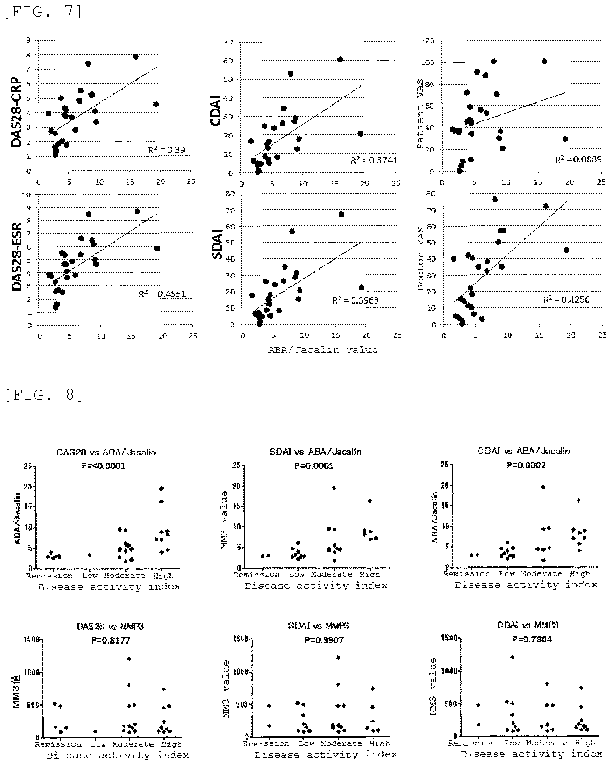

FIG. 7 shows correlation diagrams of an ABA/Jacalin value and disease activity of rheumatoid arthritis. From the signals of ABA that is one of the lectins strongly correlating with the MMP-3 level, and the signals of Jacalin, a value of X = ABA / Jacalin, which is a glycosylation discrimination index, is plotted on the horizontal axis, a value of various parameters, which is said to show disease activity of rheumatoid arthritis, is plotted on the vertical axis, and the correlation between both values was examined. As a result, some degree of correlation with all the parameters except for the value of patient VAS was observed.

FIG. 8 shows correlation diagrams of an ABA/Jacalin value and a disease activity category. Serum samples of 36 cases are classified into four categories (remission, low disease activity, moderate disease activity, and high disease activity) based on each standard of DAS28-ESR, SDAI, and CDAI, values of ABA/Jacalin are plotted, and when each distribution is examined, the numerical value of ABA/Jacalin is increased as the disease activity increases. On the other hand, no correlation was observed between the MMP-3 level and the disease activity. P values are obtained with a Spearman rank correlation coefficient.

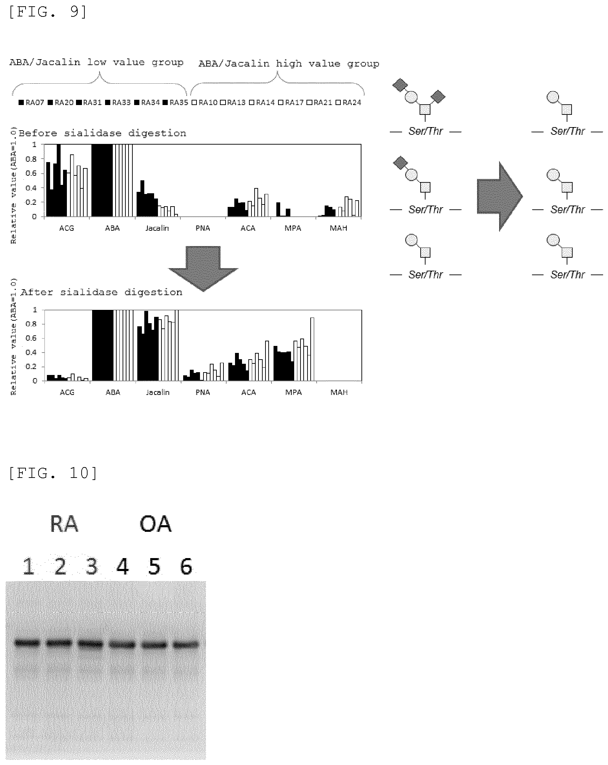

FIG. 9 shows an influence of sialidase digestion onto a glycan profile of serum-derived MMP-3. For serum samples of 12 cases, the MMP-3 was sialidase digested, and when lectin array analysis was performed including untreated ones, there was a significant difference of Jacalin signals in the samples before the digestion (see FIG. 3), but difference was eliminated in the samples after the digestion. Glycan structure changes before and after sialidase digestion, which are assumed from the results, were shown. In addition, □ represents GalNAc, ○ represents Gal, and ♦ represents NeuNAc.

FIG. 10 shows Western blotting of the MMP-3 in a synovial fluid. An MMP-3 level in each synovial fluid collected from three cases of patients affected with each of rheumatoid arthritis (RA) and osteoarthritis (OA) is measured, and MMP-3 in each of diluted synovial fluid sample which had been adjusted to a concentration of 1 μg/mL in advance was immunoprecipitated (IP) with an anti-MMP-3 antibody. Then equal volumes of obtained eluate of the immunoprecipitate were loaded into gel, and Western blotting was performed. In any sample, almost the same degree of MMP-3 level was measured.

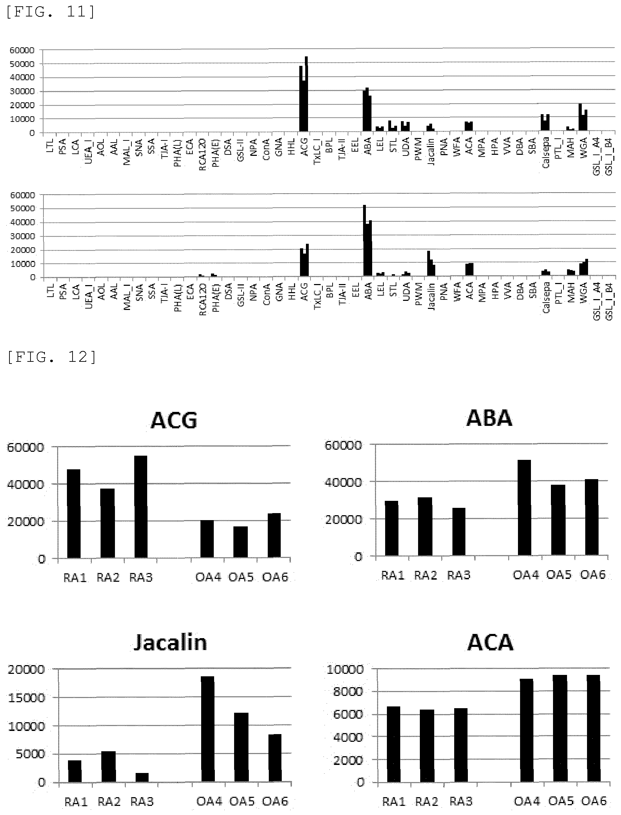

FIG. 11 shows comparative glycan analysis of MMP-3 derived fromasynovial fluid. The MMP-3 in each synovial fluid collected from three cases of patients affected with each of rheumatoid arthritis (RA) and osteoarthritis (OA) was subjected to comparative glycan analysis by a lectin array. The upper part shows a glycan profile of a sample derived from a synovial fluid of each of three cases of patients with rheumatoid arthritis, and the lower part shows a glycan profile of a sample derived from a synovial fluid of each of three cases of patients with osteoarthritis. In the present experiment, the same amount of MMP-3 was used, therefore, the signal difference means qualitative difference between glycans of MMP-3. The MMP-3 glycan profile in a synovial fluid sample derived from a patient with rheumatoid arthritis is similar to the MMP-3 glycan profile obtained by a serum sample of a patient having particularly high disease activity among the patients with rheumatoid arthritis, and has characteristics of a relatively low Jacalin value also in a synovial fluid sample. This means that a glycan structure of a Jacalin-binding O-linked glycan epitope of MMP-3, which had been produced in a synovial tissue, remains as it is with the same glycan structure even though the epitope is secreted in serum, therefore, it was shown that with a blood sample (serum sample) that has less burden on a patient, the glycan epitope analysis can be performed with almost the same degree of accuracy as that of a synovial fluid sample.

FIG. 12 shows comparison data of four lectin signals in a synovial fluid. As to the ACG, ABA, ACA, and Jacalin which were recognized to have significant signal intensity in a serum sample of a patient with rheumatoid arthritis, among the glycan profiles in FIG. 11, numerical values between the synovial fluid samples of rheumatoid arthritis (RA) and osteoarthritis (OA) were graphed. In all of the four lectins, significant difference was recognized between RA and OA, and a tendency that the significant difference between RA and OA was recognized particularly in Jacalin, and the Jacalin value was low in RA was observed. That is, it was demonstrated that the MMP-3 glycan epitope analysis in a serum sample accurately reflects the state of the MMP-3 glycan epitope in a synovial fluid.

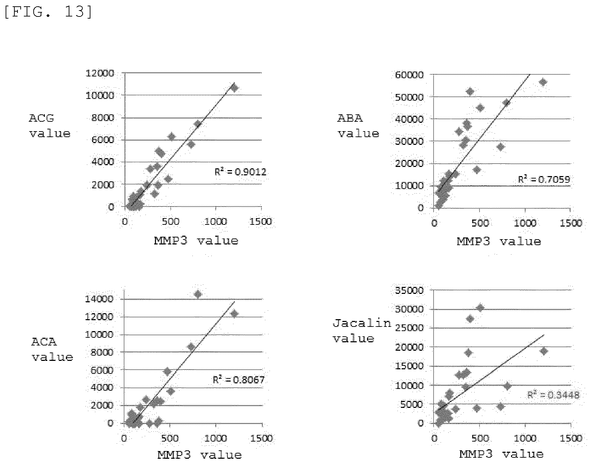

FIG. 13 shows correlation diagrams of the MMP-3 level in serum and the signal intensity of each of ABA, ACA, ACG, and Jacalin by using serum samples from a group of 29 patients in total in other cases (rheumatoid arthritis: RA, polymyalgia rheumatica: PMR, and relapsing polychondritis: RP). As in (FIG. 5), all of the signals of ABA, ACA, and ACG are correlated with the MMP-3 level, but no correlation was observed between the Jacalin signal and the MMP-3 level.

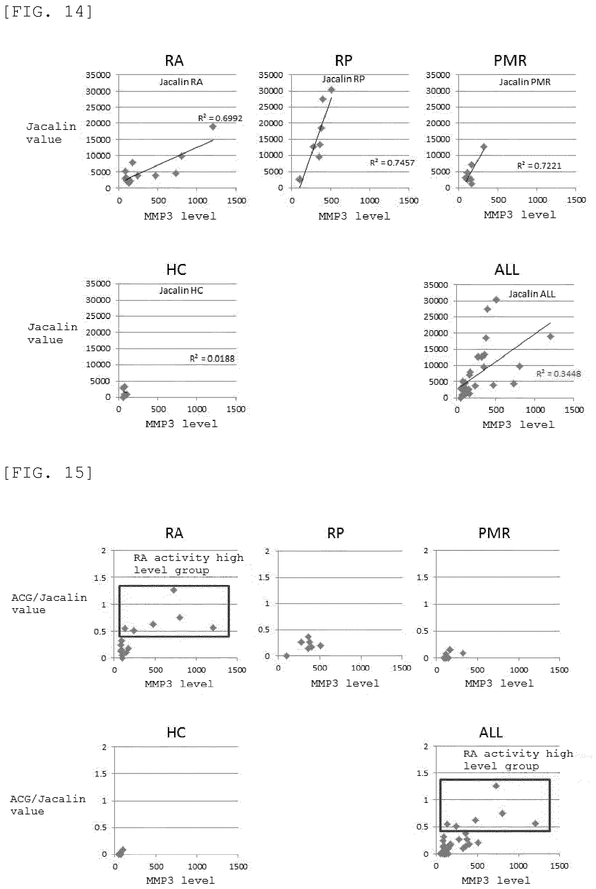

FIG. 14 shows graphs in which Jacalin values and MMP-3 levels are plotted separately for each RA, PMR, RP and healthy control subjects (HC). In RA, no correlation between the MMP-3 level and the Jacalin value was observed, and as in the divergence examples of FIG. 5, it can be seen that the Jacalin value decreases as the MMP-3 level increases, but such a tendency is not observed in non-RA, and the correlation between the Jacalin and the MMP-3 level was high.

FIG. 15 shows graphs in which the ACG/Jacalin values and the MMP-3 levels are plotted separately for each RA, PMR, RP and HC. It can be seen that only in the rheumatoid arthritis (RA), the ACG/Jacalin values are high in a group with high disease activity of RA.

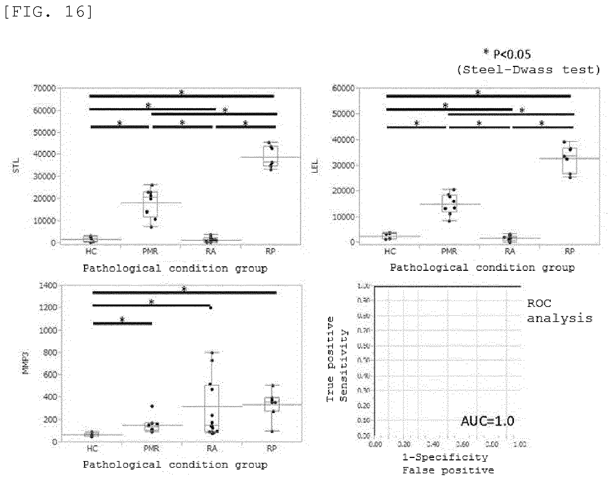

FIG. 16 shows graphs in which the STL and LEL lectin signals were measured separately for each RA, PMR, RP, andHC, and Wilcoxon test and ROC analysis were performed. In polymyalgia rheumatica (PMR) and relapsing polychondritis (RP), AUC = 1.0, and it was shown that the discrimination can be specifically performed also in both diseases.

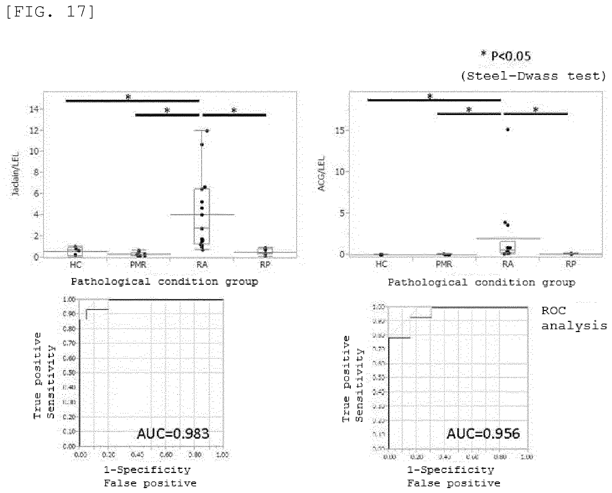

FIG. 17 shows graphs in which the Jacalin/LEL values and the ACG/LEL values were measured separately for each RA, PMR, RP, and HC, and Wilcoxon test and ROC analysis were performed. In rheumatoid arthritis (RA), in a case of the Jacalin/LEL value, AUC = 0.983, in a case of the ACG/LEL value, AUC = 0.956, and it was shown that the discrimination can be specifically performed also in any values.

Modes for Carrying Out the Invention

1. Glycan biomarker for rheumatoid arthritis in the present invention

In the present invention, the glycan utilized as an inverse correlation (negative) glycan biomarker for diagnosis of rheumatoid arthritis is a glycan of MMP-3(matrix metalloproteinases-3) derived from a synovial tissue capable of being observed in serum, and is a Jacalin-binding glycan that is seemed to be gradually lost as the disease activity of rheumatoid arthritis increases.

The value obtained by correcting the Jacalin-binding glycan amount with the abundance of MMP-3 in serum or the amount of MMP-3 glycan present constantly, or the binding amount of the glycan-recognizing probe represented by a lectin showing reactivity and correlated with the abundance of MMP-3 is a glycan biomarker amount of the present invention.

(1-1) As to MMP-3

MMP-3 is produced in a large amount in synovial cells of rheumatoid arthritis, and known to be strongly involved in the progress of cartilage destruction (Non-Patent Documents 1 to 3).

Conventionally, serums CRP and ESR that have been used as an inflammatory marker for diagnosis of rheumatoid arthritis reflect the systemic inflammation, in contrast, it is said that the MMP-3 level in serum reflects pathological conditions of local joints, and currently, is attracting attention as the most reliable prognostic factor for joint destruction. Further, early rheumatoid arthritis is difficult to be discriminated from other articular diseases such as osteoarthritis, but by the MMP-3 level in serum, which reflects the degree of cartilage destruction, rheumatoid arthritis can be diagnosed in the initial stage.

However, the correlation with the "DAS28"an index of disease activity of rheumatoid arthritis is not so high, therefore, it is unreasonable to use the MMP-3 level in serum alone as a diagnostic marker that becomes an index of the pathological conditions of rheumatoid arthritis, and the MMP-3 level in serum is clinically used in combination with the "DAS28" and the like for early diagnosis of rheumatoid arthritis, determination of therapeutic strategy, and the like.

The MMP-3 is secreted as a secretory form (molecular weight of 57, 000 or 59, 000) fromchondrocytes or synovial cells, becomes an active form (molecular weight of 45, 000, or 28, 000) in which signal peptides and the like have been removed. The MMP-3 is a metalloprotease that degrades cartilage proteoglycan constituting basement membranes and cartilages, type III, type IV, type V, type VI I, and type IX collagens, laminin, fibronectin, and gelatin (Non-Patent Documents 2, and 15).

The signal sequence and pro-sequence are removed from the MMP-3, and the MMP-3 are activated as the mature MMP-3. It is considered that the MMP-3 have, as with other MMP family members, a domain structure in which an enzyme active domain (binding domain) that binds to Zn, and a hemopexin-like domain involved in substrate recognition are connected with a hinge region (linker domain) of 75 amino acid residues or less. The glycan structure and addition position information of MMP-3 glycan are poor, and it is known that with regard to N-linked glycan, the N-linked glycan is present in an enzyme active domain. However, there is no findings regarding the O-linked glycan at all. When predicted based on the findings of other MMPs, it is considered that the O-linked glycan of MMP-3 binding to Jacalin, ABA, and ACA lectins, in which changes of the glycan structure are observed in a synovial tissue and in serum after affection with rheumatoid arthritis, is present in multiple numbers on a hinge region of MMP-3, and the O-linked glycan may also be present in other regions.

(1-2) Glycan biomarker binding lectin for rheumatoid arthritis in the present invention

In the present invention, as a negative glycan biomarker, the rheumatoid arthritis marker capable of specifically determining the disease activity of rheumatoid arthritis is a "lectin Jacalin-binding O-linked glycan of MMP-3 derived from a synovial tissue". In addition, the term "lectin" is defined as a "protein that specifically recognizes and binds to a glycan, and forms cross linkage". Each of the Jacalin, ABA, ACA, and ACG used in the present invention is one kind of lectins.

The "Jacalin", which is a lectin recognizing the glycan biomarker of the present invention, is a lectin isolated from a seed of a jackfruit (Botanical Name: *Artocarpas integliforia*), and belongs to a Jacalin-related Lectin family. It is known that it has monosaccharide specificity to Gal(from lectin frontier database (LfDB)).

The Jacalin can be extracted and purified from seeds of a jackfruit after crushing the seeds, and can be mass-produced from an E. coli host, or the like by a gene recombination technique using known nucleotide sequences. In Examples of the present invention, Unconjugated Jacalin (Catalog Number: L-1150), and Biotinylated Jacalin (Catalog Number: B-1155), which are manufactured by Vector Laboratories, or a lectin array (Product Name: "LecChip (trademark) (manufactured by GlycoTechnica Ltd.)") containing Jacalin was used.

Further, among other lectins used for analysis of the disease activity of rheumatoid arthritis for the diagnosis of rheumatoid arthritis, ABA is a lectin isolated from a mushroom (Botanical Name: *Agaricus bisporus*) and also a lectin having specificity to Gal and GlcNAc, ACA is a lectin isolated from Amaranthus of the family Amaranthaceae (Botanical Name: *Amaranthus*) and also a lectin having specificity to GalNAc, and ACG is a lectin isolated from Agrocybe cylindracea (Botanical Name: *Agrocybe cylindracea*) and belongs to the galectin family (from lectin frontier database (LfDB)).

All of the ABA, ACA, and ACG can also be extracted from the plants or mushrooms from which ABA, ACA, and ACG are derived, or can be synthesized by utilizing a gene recombination technique from each nucleotide sequence information (each accession number of database). ABA is commercially available from Wako Pure Chemical Industries, Ltd. (recombinant), Microbiologics, Inc. (naturally-derived product), or the like, ACA is commercially available from Vector Laboratories (naturally-derived product), or the like, and recombinant ACG is commercially available from Wako Pure Chemical Industries, Ltd. , or the like. A lectin array in which these ABA, ACA, and ACG are arranged is also commercially available as product name "LecChip (trademark)" from GlycoTechnica Ltd. In Examples of the present invention, "LecChip (trademark)" is used.

(1-3) As to glycan epitope to become glycan biomarker of the present invention

The glycan epitope to become a negative glycan biomarker for the diagnosis of rheumatoid arthritis of the present invention is a Jacalin-binding O-linked glycan of MMP-3. In addition, it is considered that the O-linked glycan is present in multiple numbers on a hinge region (linker domain) that connects an enzyme active domain (binding domain), and a hemopexin-like domain of MMP-3. It is also considered that the presence of the multiple O-linked glycans causes a large difference in the reactivity among the lectins.

Further, Jacalin has a ligand of "Gal ($\beta$1, 3) GalNAc" that is a core structure-1 (Core 1) of the O-linked glycan binding to a Ser/Thr residue in a peptide chain and a ligand of Tn antigen (GalNAc), and it is known that by introducing the modification of sialic acid (NeuNAc) at the 6-position of the GalNAc, the recognition is canceled. That is, it is considered that the decrease of the Jacalin signal level corresponds to the state in which a rate of sialylation at the 6-position of "GalNAc" of the above-described glycan backbone is increased. Furthermore, Jacalin is a representative example in which the binding ability is decreased by $\alpha$2, 6 sialic acid modification to GalNAc, and a lectin, a glycan-recognizing antibody, a glycan-recognizing peptide, or a glycan-recognizing aptamer, which has the similar characteristics, can be substituted.

In addition, considering that also signals of ABA and ACA that can recognize or accept a sialic acid residue modifying both of "Gal" and "GalNAc" of Core 1 are consistent with the behavior of the MMP-3 levels, in the O-linked glycan in a linker domain of MMP-3 of a healthy subject, according to the lectin array analysis data by the inventors, Core 1 is a mother skeleton, and only the Gal of or both of "Gal" and "GalNAc" are modified by sialic acid (NeuNAc), and in the affection with rheumatoid arthritis, the introduction rate of sialic acid (NeuNAc) is gradually increased depending on the increase of the disease activity of rheumatoid arthritis, and the amount of the "GalNAc" without modification at the 6-position is lost.

According to the results of the sialic acid cleavage experiment by sialidase for MMP-3 (FIG. 9), the major O-linked glycan structures possessed by MMP-3 are a core 1 structure (Gal ($\beta$1, 3) GalNAc), and it is understood that the modification of $\alpha$2, 3 sialic acid to Gal is almost accomplished. Further, it is concluded that the degree of the $\alpha$2, 6 sialylation to GalNAc is low frequency in a healthy subject, but the degree of the modification is increased as the disease activity of rheumatoid arthritis increases.

According to this, it is demonstrated that the degree of the $\alpha$2,6 sialylation to GalNAc in a core 1 structure becomes a marker of the disease activity of rheumatoid arthritis, the Jacalin-derived signals become a negative activity biomarker, and further together with the ABA and ACA-derived signals, the ACG-derived signals also show the similar behavior to that of the MMP-3 level in serum.

That is, the glycan epitope to become a biomarker for determination of disease activity of rheumatoid arthritis of the present invention can be expressed as follows:

a "glycan epitope, which is an O-linked glycan of MMP-3, and is characterized by being a Jacalin non-binding O-linked glycan epitope", or

a "glycan epitope, which is an O-linked glycan of MMP-3, and is characterized in that GalNAc binding to a Ser/Thr residue in a peptide chain of the MMP-3 is modified with $\alpha2,6$ sialic acid".

Further, this is not limited to Jacalin, and indicates that as long as being a substance that recognizes a glycan in which $\alpha$GalNAc binding to a Ser/Thr residue of MMP-3 is contained, but does not recognize a glycan in which $\alpha2$, 6 6 sialic acid binds to the $\alpha$GalNAc, the substance can be used as a reagent for determination of disease activity of rheumatoid arthritis.

Specifically, a binding lectin, a glycan-recognizing antibody, a glycan-recognizing peptide, a glycan-recognizing aptamer, and the like, which recognize the $\alpha2$, 6 sialylation to GalNAc binding to a Ser/Thr residue in a peptide chain of MMP-3 can be used. In contrast, a substance, which recognizes a glycan in which $\alpha$GalNAc binding to a Ser/Thr residue of MMP-3 is contained, but does not recognize a glycan in which $\alpha2$, 6 6 sialic acid binds to the $\alpha$GalNAc, that is, a substance selected from a glycan-recognizing antibody, a binding lectin, a glycan-recognizing peptide, and a glycan-recognizing aptamer, which recognizes a glycan in which $\alpha$GalNAc binding to a Ser/Thr residue is contained, but does not recognize a glycan in which $\alpha2,6$ sialic acid binds to the $\alpha$GalNAc, can also be used as a reagent for determination of disease activity of rheumatoid arthritis. The latter typical example is Jacalin used in the present Examples.

2. Method for determining disease activity of rheumatoid arthritis of the present invention

(2-1) Biomarker for determination of disease activity of rheumatoid arthritis of the present invention

The present invention is characterized in that in order to determine the disease activity of rheumatoid arthritis, changes in a glycan structure of an O-linked glycan of MMP-3 in a blood sample is measured.

For this purpose, the positive glycan epitope to become a biomarker for determination of rheumatoid arthritis activity can be expressed as follows:

a "glycan epitope, which is an O-linked glycan of MMP-3, and is characterized by being a Jacalin non-binding O-linked glycan epitope", or

a "glycan epitope, which is an O-linked glycan of MMP-3, and is characterized in that GalNAc binding to a Ser/Thr residue in a peptide chain of the MMP-3 is modified with $\alpha2,6$ sialic acid".

Further, by detecting and quantifying the glycan epitope, the presence or absence of the affection with rheumatoid arthritis, and the degree of disease activity of rheumatoid arthritis can be determined.

A substance specifically recognizing the glycan epitope, that is, a substance selected from a glycan-recognizing antibody, a binding lectin, a glycan-recognizing peptide, and a glycan-recognizing aptamer, which specifically recognizes the O-linked glycan in which GalNAc binding to a Ser/Thr residue is modified with $\alpha2,6$ sialic acid, becomes a reagent for determination of disease activity of rheumatoid arthritis to detect and quantify the glycan epitope.

In contrast, a substance, which recognizes a glycan in which $\alpha$GalNAc binding to a Ser/Thr residue of MMP-3 is contained, but does not recognize a glycan in which $\alpha2$, 6 6 sialic acid binds to the $\alpha$GalNAc, that is, a substance selected from a glycan-recognizing antibody, a binding lectin, a glycan-recognizing peptide, and a glycan-recognizing aptamer, which recognizes a glycan in which $\alpha$GalNAc binding to a Ser/Thr residue is contained, but does not recognize a glycan in which $\alpha2,6$ sialic acid binds to the $\alpha$GalNAc, can also be used as a reagent for determination of disease activity of rheumatoid arthritis. The latter typical example is Jacalin used in the present Examples. In the following analysis method, explanation will be made by using mainly Jacalin, but it is not limited to the Jacalin.

Further, the MMP-3 itself, which is fractionated from a body fluid sample such as a blood sample, and has multiple Jacalin non-binding O-linked glycans, that is, the MMP-3 itself, which has multiple O-linked glycans in which GalNAc binding to a Ser/Thr residue in a peptide chain is modified with $\alpha2$, 6 sialic acid, becomes a marker for determination of rheumatoid arthritis activity.

In order to detect or quantify such an MMP-3, an analysis method similar to that in the above-described glycan epitope is applied.

Further, in order to determine the disease activity of rheumatoid arthritis in a body fluid sample such as a blood sample, in measuring the changes in a glycan structure, the measurement can be performed by calculating the following glycosylation discrimination index X, which is obtained by multiplying the measured signals corresponding to the total amount of MMP-3 in a body fluid sample by the reciprocal of measured signals corresponding to the total amount of Jacalin-binding O-linked glycan of MMP-3 in said body fluid sample, or glycosylation discrimination index Y, which is obtained by multiplying the reciprocal of measured signals corresponding to the total amount of MMP-3 in a body fluid sample by the measured signals corresponding to the total amount of Jacalin-binding O-linked glycan of MMP-3 in said body fluid sample.

Further, the glycosylation discrimination index (X or Y) can be defined as an "index for enabling the comparative evaluation of the state of modification on the mother skeleton by combining signals and making mathematical formulas in a case where the strength of this binding can be converted as quantifiable signals, provided that in a case where there are multiple lectin groups having similar specificity, and in particular, the composition (for example, sialic acid, fucose, or the like), the manner (to which glycan and at which position), and the proportions of the modification to a certain glycan structure of a mother skeleton (for example, the core 1 structure in the present invention) differ, the strength of binding of each lectin differs.

```
X = measured signals corresponding to the total amount
of MMP-3 in a body fluid sample / measured signals corresponding
to the total amount of Jacalin-binding O-linked glycan on the
MMP-3 in said body fluid sample,
```

and

```
Y = measured signals corresponding to the total amount
of Jacalin-binding O-linked glycan of MMP-3 in a body fluid
sample/ measured signals corresponding to the total amount of
the MMP-3 in said body fluid sample.
```

At that time, as the X value of the glycosylation discrimination index is higher, the disease activity of rheumatoid arthritis can be determined to be higher, in contrast, as the Y value of the glycosylation discrimination index is higher, the disease activity of rheumatoid arthritis can be determined to be lower.

In order to determine whether or not the subject is suffering from rheumatoid arthritis, or to determine the disease activity of rheumatoid arthritis at a specific time, it is preferred to provide a process in which a reference value $(X_0)$ or $(Y_0)$ in a healthy subject is set, and the X and Y measured and calculated with a body fluid sample derived from a subject are compared.

When the X is higher than the reference value $X_0$ $(X > X_0)$, it is determined that the subject has high disease activity of rheumatoid arthritis, or is affected with rheumatoid arthritis; or when the Y is lower than the reference value $Y_0$ $(Y < Y_0)$, it is determined that the subject has high disease activity of rheumatoid arthritis, or is affected with rheumatoid arthritis.

By observing a subject over time, condition changes over time of the disease activity of rheumatoid arthritis of the subject can be determined.

In that case, it is preferred to provide a process in which X' and Y' measured and calculated with a body fluid sample derived from a subject are compared with a value $(X'_0)$ or $(Y'_0)$ calculated for a body liquid sample before the test day from the same subject. In a case of X' being a numerical value higher than the value before the test day $(X' > X'_0)$, and in a case of Y' being a numerical value lower than the value before the test day $(Y' < Y'_0)$, it is determined that the disease activity of rheumatoid arthritis of a subject is deteriorated, and in a case of being a lower numerical value $(X' < X'_0)$ or a case of being a higher numerical value $(Y' > Y'_0)$, it is determined that the disease activity is improved.

By comparing a body fluid sample derived from a subject after the rheumatoid arthritis treatment with a body fluid sample before the treatment, the therapeutic effect can be determined.

Herein, as "measured signals corresponding to the total amount of MMP-3" in a body fluid sample, a measurement value of the abundance of MMP-3 in a body fluid sample, or measurement signals increasing or decreasing depending on the abundance of MMP-3 in a body liquid sample can be used.

The expression "measurement value of the abundance of MMP-3 in a body fluid sample" is referred to as a measurement value of an absolute value of the abundance of MMP-3 contained in a body fluid sample, and for those skilled in the art, the method for measuring the abundance of MMP-3 in a sample is known. Specifically, a latex agglutination method using monoclonal antibodies against MMP-3, which are coated on latex particles, or the like can be used.

Further, as the "measurement signals increasing or decreasing depending on the abundance of MMP-3" in a body fluid sample, in addition to a signal level measured by using an anti-MMP-3 antibody (antibody specifically binding to a polypeptide chain of MMP-3), a measurement signal level showing a signal amount derived from the ABA, ACA, or ACG showing the same behavior as that of the MMP-3 level, that is, a binding amount of the MMP-3 and the ABA, ACA, and ACG, in a body fluid sample is preferably used. In addition, not only the ABA, ACA, and ACG, but also a lectin recognized to be correlated with the MMP-3 level can be substituted.

The most preferable X and Y of the present invention are as follows.

$$X = ABA\text{-}derived\ measurement\ signals\ in\ a\ body\ fluid\ sample/$$

$$Jacalin\text{-}derived\ measurement\ signals\ in\ said\ body\ fluid\ sample,$$

and

$$Y = Jacalin\text{-}derived\ measurement\ signals\ in\ a\ body\ fluid$$

$$sample/\ ABA\text{-}derived\ measurement\ signals\ in\ said\ body\ fluid$$

$$sample.$$

(2-2) Detection and quantification by lectin array or sandwich ELISA method

In the present invention, by measuring a Jacalin signal level of a test sample using a lectin array or a sandwich ELISA method, and by correcting the reciprocal of the Jacalin signal level with the ABA, ACA or ACG signal level measured at the same time, the presence or absence of the affection with rheumatoid arthritis, or the disease activity of rheumatoid arthritis can be determined. The ABA, ACA, or ACG signal level can be replaced with the MMP-3 level.

The lectin array can be analyzed either by a direct method in which MMP-3 is fluorescent-labeled and applied to a lectin array, and after the binding reaction, signals are detected, or by an indirect method in which unlabeled MMP-3 is applied to a lectin array, and then binding signals are detected by an antibody-overlay method.

Herein, as a test sample, not only the serum or blood plasma obtained from the blood collected from a subject, but also other body fluid samples such as joint fluid (synovial fluid), pleural effusion, ascites, synovial membrane fluid, and lymph, can be targeted. The subject is not particularly limited, and those methods can be widely applied to any person who needs to be determined whether or not affected with the rheumatoid arthritis, or determined to have the disease activity of rheumatoid arthritis.

In addition, it is most preferred to use blood (serum, plasma, or the like) of a subject as a test sample because the burden on a subject is low, and the shortened test time can be achieved.

According to the detection method of the present invention, the decrease of glycan biomarkers for rheumatoid arthritis is detected in a test sample, and the disease activity of rheumatoid arthritis can be accurately evaluated. Further, the effect of rheumatoid arthritis treatment is accurately determined, and the degree of remission can be determined.

(2-3) Lectin array analysis method

<Concentration of MMP-3-containing fraction>

The Jacalin-binding glycan to become a (negative) glycan biomarker for rheumatoid arthritis with an inverse correlation of the present invention is a glycan of MMP-3, therefore, in a case where the amount of a test sample such as a blood sample of a subject is a minute amount, and the like, by using an MMP-3 concentrated fraction by an affinity column or the like using an anti-MMP-3 antibody, more accurate determination of the disease activity of rheumatoid arthritis can be achieved.

Further, following a purification method using the streptavidin-coated magnetic beads described in WO 2011/007797, also by biotinylating the anti-MMP-3 antibody, and using the magnetic beads conjugated to streptavidin - coated magnetic beads, the concentrate can be efficiently performed. In Examples of the present invention, by using the magnetic beads, the MMP-3 concentrated fraction is obtained.

<Labeling>

Examples of the labeling substance include a fluorescent substance (for example, FITC, rhodamine, Cy-3, and Cy-5), a radioactive substance (for example, $^{14}$C, and $^{3}$H), and an enzyme (for example, alkaline phosphatase, peroxidase (horseradish peroxidase, and the like), glucose oxidase, β-galactosidase). Further, the binding between biotin and (strept) avidin can also be utilized. The detection agent is labeled with biotin, the (strept)avidin is labeled with the above-described labeling substance, and by utilizing the binding between the biotin and the (strept)avidin, the detection can also be performed.

In a case of using an enzyme as a labeling substance, the detection is performed by using an appropriate substrate depending on the enzyme to be used. For example, in a case of using peroxidase as an enzyme, o-phenylenediamine (OPD), tetramethylbenzidine (TMB), or the like is used as the substrate, and in a case of using alkaline phosphatase, p-nitrophenyl phosphate (PNPP), or the like is used. As to also the enzyme reaction stop solution, and the substrate solution, depending on the selected enzyme, any conventionally known ones can be appropriately selected and used.

<Preparation of lectin array>

As a lectin array, any lectin array can be used as long as the lectin array contains a lectin of any one of ABA, ACA, and ACG, together with a Jacalin lectin. For example, a lectin array in which 45 kinds of plant lectins having different specificities are immobilized on the same substrate, which has been developed by the present inventors (Non-Patent Document 16), or LecChip (trademark) Ver.1.0 (manufactured by GlycoTechnica Ltd.) can be used. However, the lectin array can be prepared appropriately according to a known method.

As the lectin array, a lectin array containing only a lectin of any one of ABA, ACA, and ACG, together with a Jacalin lectin can be used, but a lectin array containing all of the ABA, ACA, and ACG lectins is preferred, and a lectin array in which multiple other lectins are immobilized on a substrate is more preferred. Examples of the other lectins at that time include a MAH lectin, a MAL lectin, and a WGA lectin.

Jacalin, and other lectins may be immobilized directly on a substrate (direct method), but Jacalin and the like are biotinylated to obtain biotinylated Jacalin and the like, and by preparing a lectin array in a form immobilizing the biotinylated Jacalin and the like on a streptavidin-coated substrate (indirect method), improvement of detection sensitivity, and decrease of background can be drastically promoted.

As the substrate of a lectin array, any transparent material can be used as long as evanescent waves can penetrate the material, and a synthetic resin such as slide glass, and polycarbonate, or the like is generally used.

<Application to lectin array and washing>

A test sample labeled with Cy-3 or the like, which is diluted with a buffer or undiluted, is applied into a lectin array reaction vessel, and allowed to interact with each other, and then Non-specifically binding contaminants are washed with a buffer for a lectin array (available on the market). In addition, the washing process can be omitted in a case of detecting with evanescent waves.

<Detection method>

In general, the binding of a lectin to a glycan is generally weaker compared with the binding of antigen-antibody reaction, and the binding constant of antigen-antibody reaction is around $10^{6}$ to $10^{9}$ M$^{-1}$, but in contrast, the binding constant between a lectin and a glycan is $10^{4}$ to $10^{7}$ M$^{-1}$, therefore, in the detection of the glycan binding signals of a lectin, usually an evanescent wave excitation fluorescence detect ion method is preferably used, and also in the present invention, an evanescent wave excitation fluorescence detection method is preferably used. The evanescent wave excitation fluorescence detection method is a method utilizing the fact that when light is made incident under the conditions such that total reflection occurs on the end face (side face) of a slide glass, in a case of two phases having different refractive indexes, such as glass (solid phase) and water (liquid phase), light in extremely short range called "evanescent waves" (called near-field light) oozes out only in the near field of around several hundred nm from the interface. According to this method, excitation light for a fluorescent substance is incident from the end face, and only the fluorescent substance existing in the near field is excited, and fluorescence observation is performed. The evanescent wave excitation fluorescence detection method is described in Non-Patent Document 16, or the like. In this detection, GlycoStation (trademark) Reader 1200 (manufactured by MORITEX Corporation, and GlycoTechnica Ltd.), or the like can be used.

However, it shows a result that in the binding ability between a lectin such as Jacalin to be a detection target in the present invention and an O-linked glycan epitope of MMP-3, according to analysis results in Examples, an extremely high binding constant is estimated. This is considered to be a cluster effect due to the presence of multiple O-linked glycans that are assumed to be present in a hinge region of MMP-3. It is said that the interaction between the lectin with high density immobilized on a substrate, and the glycan has 2 digits or more of effects in binding constant due to the cluster effect. In addition, it is considered that the cluster effect is said in particular to have an effect of remarkably slowing the dissociation rate, therefore, the detection is also possible in a detection system requiring a washing process. Accordingly, an antibody-overlay-lectin array method that is an indirect method containing a washing process, or as described later, a general detection method as used in a detection system of antigen-antibody reaction, such as a sandwich ELISA method can be applied.

<Detection method>

The evaluation by a lectin array is performed as follows. An ABA lectin or the like, which is a lectin recognizing glycans present at a substantially constant ratio in a body fluid, for example, serum-derivedMMP-3, or correlating with the MMP-3 level and the binding signal level is selected as an internal standard lectin, the selected ones are immobilized on the same lectin array substrate, the target lectin signals such as Jacalin signals are converted to a relative value, and then determination of exceeding or not exceeding a certain cutoff value is made. As described above, a method in which based on the level of a certain lectin, signals of the target lectin are converted into a relative value and used for discrimination can be performed according to a literature (Non-Patent Document 17) previously published by the present inventors. The setting of the cutoff value can be performed in advance using a body fluid, for example, serum, sampled f rommultiple patients with rheumatoid arthritis. That is, based on the above-described relative values obtained in advance from the lectin array analysis targeting the serum derived from multiple patient with rheumatoid arthritis and the serum of healthy subjects, the glycosylation discrimination index (X or Y) described later is calculated. More preferably, multiple glycosylation discrimination indexes corresponding to the degree of disease activity of rheumatoid arthritis are prepared in advance, and as compared with the glycosylation discrimination index derived from a blood sample, the degree of the disease activity of rheumatoid arthritis of a subject is determined, and also it is determined whether or not the subj ect is affected with rheumatoid arthritis, or how the degree of the disease activity of rheumatoid arthritis is.

(2-4) Sandwich method

In a sandwich method, any of an anti-MMP-3 antibody, and a lectin such as Jacalin is allowed to bind to a solid phase. Hereinafter, the immobilized binding substance is referred to as a "capture agent", and the other is referred to as a "detection agent". Examples of the substrate (solid phase) to immobilize the capture agent include a plate (for example, microwell plate), a microarray substrate (for example, slide glass for microarray), a tube, beads (for example, plastic beads, and magnetic beads), a carrier for chromatography (for example, Sepharose (trademark)), a membrane (for example, nitrocellulose membrane, and PVDF membrane), and a gel (for example, polyacrylamide gel) . Among them, a plate, beads, and a membrane are preferably used, and a plate is most preferably used because of the ease of the handling. The capture agent may be immobilized by any method as long as the sufficient binding strength is obtained, and is immobilized by, for example, covalent bonding, ionic bonding, physical adsorption, or the like. Alternatively, a substrate in which a capture agent is immobilized in advance may be used.

The detection agent may be indirectly or directly labeled with a labeling substance. Examples of the labeling substance include a fluorescent substance (for example, FITC, rhodamine, Cy-3, and Cy-5), a radioactive substance (for example, $^{13}$C, and $^{3}$H), and an enzyme (forexample, alkaline phosphatase, peroxidase (horseradish peroxidase, and the like), glucose oxidase, β-galactosidase). Further, the detection agent is labeled with biotin, the (strept) avidin is labeled with the above-described labeling substance, and the binding between the biotin and the (strept)avidin may also be utilized.

In a case of using an enzyme as a labeling substance, the detection is performed by using an appropriate substrate depending on the enzyme to be used. For example, in a case of using peroxidase as an enzyme, as the substrate, o-phenylenediamine (OPD), tetramethylbenzidine (TMB), or the like is used, and in a case of using alkaline phosphatase, p-nitrophenyl phosphate (PNPP), or the like is used. As to also the enzyme reaction stop solution, and the substrate solution, depending on the selected enzyme, any conventionally known ones can be appropriately selected and used.

The capture agent forms a complex with the MMP-3 in a blood sample or with the Jacalin, ABA, ACA andACG lectin-binding glycan of MMP-3. By measuring the signals generated by applying a detection agent to the complex, the Jacalin, ABA, ACA, and ACG lectin-binding glycans of MMP-3 in serum are detected and quantified. The measurement of the signals may be performed by using an appropriate measuring apparatus depending on the labeling substance used.

As to the sandwichmethod for measuring the Jacalin-binding glycan epitope of the present invention, specifically, by immobilizing multiple lectins containing Jacalin on a substrate, adding a test sample, and performing the reaction, the MMP-3 binding to the lectin is presented on the reaction field, to which the labeled MMP-3 binding substance is allowed to react, and the detection can be performed. The multiple lectins to be used can be aligned (arrayed) on the same substrate, or can be immobilized on different substrates and parallelly measured. At that time, in place of the method (direct method) that is performed by immobilizing a lectin such as Jacalin directly on a substrate, a method (indirect method) in which each of the lectins of Jacalin and the like is biotinylated, and the biotinylated lectins are prepared in a form of being immobilized on a streptavidin-coated substrate is used, and improvement of detection sensitivity, and decrease of background can be drastically promoted.

In another method, by immobilizing an MMP-3 binding substance on a substrate, adding a test sample, and performing the reaction, MMP-3 is prepared in a form of overlaying the MMP-3 on the binding substance, and then by a labeled Jacalin lectin and other labeled lectins, the MMP-3 showing reactivity to the lectins can be detected.

For the measurement of the Jacalin-binding glycan content of the present invention, ELISA, immunochromatography, radioimmunoassay (RIA), fluorescence immunoassay (FIA method), chemiluminescent immunoassay, evanescent wave analysis method, or the like can be used. These methods are well known to those skilled in the art, and any method may be selected. Further, these methods may be performed in accordance with ordinary procedures, and setting of actual reaction conditions and the like are within the technical scope in which those skilled in the art can usually perform. Among them, it is particularly preferred to use lectin-antibody sandwich ELISA using an anti-MMP-3 antibody as an MMP-3 binding substance.

The MMP-3 having a Jacalin-binding glycan in a body fluid sample such as a blood sample forms a complex with a lectin such as Jacalin, or an anti-MMP-3 antibody on a substrate used as a capture agent. By measuring the signals generated by applying a labeled lectin or a labeled anti-MMP-3 antibody to this complex as a detection agent, Jacalin-binding glycan in the test sample is detected and quantified. The measurement of signals may be performed by using an appropriate measuring apparatus depending on the labeling substance used.

In a case where a blood sample of a subject is used as a test sample, for example, sandwich ELISA analysis can be performed in the following procedures.

The preparation method of the test sample including the labeling is the same as that in the lectin array analysis method of (2-2).

Next, for example, biotinylated Jacalin is bound to each well of an ELISA plate coated with streptavidin, and a blood sample or the MMP-3 fraction is added and allowed to interact with the MMP-3 in the sample. Subsequently, unreacted Jacalin is blocked with a blocking agent with washing by a buffer solution or without the washing, and the labeledMMP-3 antibody is reacted.

Further, in the sandwich ELISA, it is similar to the case in the lectin array analysis that it is preferred to combine another lectin such as a lectin of ABA, ACA, or ACG other than Jacalin.

Furthermore, in place of the lectin-coated ELISA plate, an antibody-coated ELISA plate in which an anti-MMP-3 antibody is immobilized on a substrate can be used. In that case, a blood sample is added to perform an antigen-antibody reaction, and then labeled Jacalin, and another labeled lectin (ABA, ACA, or ACG) are reacted, and each signal level can be measured.

<Detection and discrimination method of rheumatoid arthritis activity marker>

An ELISA method is a well-known technique, and may be performed in accordance with ordinary procedures, and an optimum measuring device can be applied for each labeling.

In quantitative detection of rheumatoid arthritis biomarkers by the present method, by using the Jacalin-binding MMP-3, which is prepared from serum or the like and whose protein amount is known, as a standard substance, a calibration curve is created and can be converted into a substantial amount of the standard substance. Recombinant MMP-3 may be used as a standard substance as long as the binding ability to Jacalin is recognized. For example, as in Example 1, commercially available recombinant MMP-3 (product number 513-MP-010, manufactured by R&D Systems Inc.), which has been massively expressed in mouse myeloma cells (N0 cells) and purified, can be used as a standard substance. Further, in an evaluation method without using a standard substance, following the lectin array method described above, a lectin in which the signals do not vary depending on the lesion is used as an internal standard lectin, the Jacalin signals are converted to a relative value, and then determination of exceeding or not exceeding a certain cutoff value is made. In addition, selection of the internal standard lectin, and setting of the cutoff value can be performed in advance by using blood samples of a large number of the patients with rheumatoid arthritis in which the disease activity has been found. That is, in advance, from lectin array analysis that targets blood samples collected from a large number of patients having different disease activities, the internal standard can be statistically set beforehand. In addition, based on the above-described relative value obtained in advance by ELISA measurement that targets blood samples collected from a large number of patients having different disease activities, a discriminant is created. More preferably, multiple discriminants corresponding to the progress of the rheumatoid arthritis or the degree of the deterioration of disease activity are created, the progress of the rheumatoid arthritis or the degree of the deterioration of disease activity of a blood sample is determined, and also it is determined whether or not the subj ect is affected with rheumatoid arthritis, or what extent the stage of the disease activity of rheumatoid arthritis is.

For example, in a case where ABA is used as an internal standard lectin, by using the numerical values obtained by the measurement of sandwiching 40 μL each of blood samples between MMP-3-ABA and MMP-3-Jacalin, it is determined as follows: remission when the MMP-3-ABA / MMP-3-Jacalin ratio is less than 2, low disease activity when the ratio is 2 or more to less than 3, moderate disease activity when the ratio is 3 or more to less than 8, and high disease activity when the ratio is 8 or more.

In addition, the ELISA measurement can be replaced with a measurement method based on a different interaction analysis principle, such as microarray and SPR. In that case, those measurement methods are well-known techniques, therefore, may be performed in accordance with ordinary procedures, and an optimum measuring device can be applied for each technique.

3. Kit for determination of disease activity of rheumatoid arthritis

A substance, which recognizes a glycan in which αGalNAc binding to a Ser/Thr residue of MMP-3 is contained, but does not recognize a glycan in which α2, 6 sialic acid binds to the αGalNAc, preferably, Jacalin can be used as an active component of a reagent for determination of disease activity of rheumatoid arthritis.

Further, a substance specifically binding to MMP-3 in an MMP-3 abundance-dependent manner for the MMP-3 in a blood sample, specifically, in combination with a lectin selected from the group consisting of ABA, ACA, and ACG, or an anti-MMP-3 antibody, can be used as a kit for determination of disease activity of rheumatoid arthritis.

In the kit for determination of activity of the present invention, for example, the following combination can be used.

(1)

(a) A substrate in which Jacalin is contained as an essential lectin, and further preferably multiple lectins containing at least one of ABA, ACA, and ACG are bound directly or indirectly via biotin-avidin; and
(b) a labeling substance capable of labeling MMP-3 in a blood sample.

(2)

(a) A substrate in which Jacalin is contained as an essential lectin, and further preferably multiple lectins containing at least one of ABA, ACA, and ACG, which becomes internal standard lectins, are bound directly or indirectly via biotin-avidin; and
(b) a labeled anti-MMP-3 antibody.

(3)

(a) A substrate in which an anti-MMP-3 antibody is bound directly or indirectly via biotin-avidin; and
(b) multiple labeled lectins in which labeled Jacalin is contained, and further preferably at least one of ABA, ACA, and ACG is contained.

At that time, multiple lectins may be immobilized on a single substrate, but each of the lectins may be immobilized on a different substrate and used.

The reagent for diagnosis or the kit for diagnosis, of the present invention is used for a subject who wants to know whether or not being affected with rheumatoid arthritis, and in particular, used for determining whether or not being affected with early rheumatoid arthritis that shows symptoms similar to those of osteoarthritis or the like. At that time, measurement values obtained in advance by applying to healthy subj ects and/or patients with rheumatoid arthritis can be used as reference values.

Further, the reagent or the kit is used to accurately grasp the pathological changes of disease activity over time in patients with rheumatoid arthritis.

Furthermore, the reagent or the kit is used to objectively determine the effect of the treatment in patients undergoing the rheumatoid arthritis treatment. In particular, the reagent or the kit is used to determine whether or not the patient has achieved a remission, that is, whether or not the patient is no longer necessary to continue the treatment.

4. A diagnosing method for accurately discriminating similar rheumatic diseases, and diagnostic kit for the method

According to the present invention, in relation to glycan of MMP-3 in the body fluid sample (such as a serum sample) which is reactive to polylactosamine structure recognizing lectin, such as a LEL or STL lectin, it has been shown that the reactivity with a body fluid sample of a patient with relapsing polychondritis (RP) particularly among the rheumatic diseases is extremely high with a significant difference, next, the reactivity with a body fluid sample of a patient with polymyalgia rheumatica (PMR) is high with outstanding significance compared with the reactivity with body fluid samples from patients with rheumatoid arthritis (RA), and healthy control subjects (HC).

That is, a kit combining a lectin recognizing a polylactosamine structure such as a LEL lectin, and/or a STL lectin together with an anti-MMP-3 antibody becomes a kit for diagnosis of recurrent polychondritis (RP) and/or a kit for diagnosis of polymyalgia rheumatica (PMR).

Herein, it has been conventionally known that a LEL lectin is as a GlcNAc reactive lectin derived from *Lycipersicon esculentum,* and a STL lectin is as a GlcNAc reactive lectin derived from *Solanum tuberosum.* Both lectins are considered to recognize GlcNAc of a polylactosamine structure. In addition, other information on LEL lectin is described in detail in a literature of the present inventors (Methods in Enzymology, vol. 479, 2010, p. 185-204), and other information on STL lectin is described in detail also in a literature of the present inventors (Methods in Enzymology, vol. 478, 2010, p. 181-195).

Further, as other lectins recognizing a polylactosamine structure, Galectin-3 and the like are known.

As a measurement method of a LEL or STL lectin-reactive glycan of MMP-3 and the like3 in a specific body fluid sample (such as a serum sample), a method in which applying the lectin array method described in (2-3) above, and for a body fluid sample such as a serum sample, the MMP-3 concentrated fraction is measured by a lectin array that contains a lectin recognizing a polylactosamine structure, such as a LEL lectin, and/or a STL lectin can be applied.

Further, applying the sandwich method described in (2-4) above, for a body fluid sample such as a serum sample, a sandwich method using an anti-MMP-3 antibody, and a lectin recognizing a polylactosamine structure, such as a LEL lectin, and/or a STL lectin, for example, a sandwich ELISA method with a combination of a labeled anti-MMP-3 antibody, and a lectin array containing a LEL lectin, and/or a STL lectin, or the like is used.

By using the measurement methods described above, relapsing polychondritis (RP), and/or polymyalgia rheumatica (PMR) can be specifically diagnosed.

With the above-described kit combining a lectin recognizing a polylactosamine structure such as a LEL lectin, and/or a STL lectin together with an anti-MMP-3 antibody, an MMP-3 O-linked glycan recognition lectin, such as Jacalin, and ACG is further combined, as a result, a kit for diagnosis of polymyalgia rheumatica (PMR), relapsing polychondritis (RP), and rheumatoid arthritis (RA), which enables significant discrimination from rheumatic diseases similar to one another can be provided. Herein, when the expression "MMP-3 O-linked glycan recognition lectin" is used, the Jacalin, ABA, ACA and ACG in which the binding ability has been confirmed in the MMP-3 concentrated fraction in serum of the rheumatoid arthritis (RA), described in (1-2) above are indicated. In addition, alternatively, for example, a MAH lectin or the like having a sialyl core 1 structure can be used.

An accurate diagnostic method of rheumatoid arthritis (RA), and a method for discriminating from similar rheumatic diseases include a process of calculating a ratio of reactive signals with a LEL or STL lectin-reactive glycan of MMP-3 in a body fluid sample (serum sample or the like), and reactive signals with an MMP-3 O-linked glycan recognition lectin, such as Jacalin, and ACG are used.

Specifically, a method in which applying the lectin array method described in (2-3) above, for a body fluid sample such as a serum sample, the MMP-3 concentrated fraction is measured by a lectin array that contains an MMP-3 O-linked glycan recognition lectin such as Jacalin, and ACG, together with a lectin recognizing a polylactosamine structure, such as a LEL lectin, and/or a STL lectin can be applied.

Further, applying the sandwich method described in (2-4) above, for a body fluid sample such as a serum sample, a sandwich method using an anti-MMP-3 antibody, a lectin recognizing a polylactosamine structure, such as a LEL lectin, and/or a STL lectin, and further an MMP-3 O-linked glycan recognition lectin such as Jacalin, and ACG, for example, a sandwich ELISA method with a combination of a labeled anti-MMP-3 antibody, and a lectin array containing an MMP-3 O-linked glycan recognition lectin such as Jacalin, and ACG, together with a LEL lectin, and/or a STL lectin, or the like can be used.

Examples

Hereinafter, the present invention is further specifically explained showing Examples, however, the present invention should not be limited by the following Examples.

Other terms and concepts in the present invention are based on the meanings of terms conventionally used in the field, and various techniques used to perform the present invention can be easily and reliably performed by those skilled in the art based on known literatures and the like, particularly except for the techniques clearly indicated the source. Further, various types of analysis, and the like were performed according to the method described in instruction manuals, catalogs, and the like of the used analytical instruments, reagents, and kits.

In addition, the contents of the technical literatures, patent gazettes, and patent application specifications cited in the present specification shall be referred to as the description content of the present invention.

Moreover, all of the body fluids (serum, and synovial fluid) used in each Example are derived from the patients who received informed consent at the clinical institutions of the collection sources, and the study was received the ethical review, and approved in each of the institutions to which the inventors belong.

(Example 1) <u>Measurement of the abundance of MMP-3 in serum sample</u>

In the present Example, serum samples of 32 cases of rheumatoid arthritis patients and 4 cases of other arthritis patients were collected, and 30 cases among these serum samples were subjected to the measurement of the abundance of MMP-3.

Simplified fractionation and concentration (enrichment) of the MMP-3 in serum was performed based on the technique (Non-Patent Document 14) that had been developed previously by the present inventors, by optimizing the conditions for the MMP-3 proteins of which the blood concentration is extremely low compared with the proteins analyzed by the inventor so far, as follows.

(1-1) Preclear process

Among the proteins contained in serum, an operation (preclear) to remove in advance the ones nonspecifically binding to the magnetic beads to be used in an antigen-antibody reaction field was performed. That is, to 40 μL aliquot of the serum in a 1.5 mL volume tube, 10 μL of the streptavidin-immobilized magnetic beads (SA beads) solution (manufactured by Tamagawa Seiki Co., Ltd., 10 mg/mL) that had been washed three times in advance with a reaction buffer (Tris-buffered saline (TBSTx) containing 1% Triton X-100), and 36 μL of reaction buffer were added to prepare a solution, and the resultant mixture solution was reacted with shaking at 4°C for 30 minutes. After the reaction, the magnetic beads were trapped in one place with a magnet, and the resulting supernatant was collected in another 1.5 mL volume tube.

(1-2) Immunoprecipitation process

To the recovered supernatant, 4 μL of biotinylated anti-MMP-3 antibody (manufactured by R&D Systems Inc., 50 μg/mL) was added, and the resultant mixture was subjected to an antigen-antibody reaction at 4°C overnight while shaking. After the reaction, to the mixture, 10 μL of the SA bead solution (10 mg/mL) washed with a reaction buffer was added, the resultant mixture was reacted with shaking at 4°C for 30 minutes. Magnetic beads bound to an antigen-antibody complex were recovered with a magnet, the obtained supernatant was removed, and then the beads were washed three times with 200 μL of reaction buffer. At the time of recovering the beads with a magnet, the washing for completely removing the contaminant proteins which had been sandwiched between the beads and non-specifically recovered was achieved by adding a reaction buffer to the magnetic beads, and then by completely dispersing the beads using a ultrasonic dispersion device (manufactured by Tamagawa Seiki Co., Ltd.), a bug crusher (manufactured by TIETECH Co., Ltd.) that is a multi-purpose spin down mixer, or the like.

(1-3) Elution process

In order to elute the MMP-3 trapped indirectly to the beads via antibodies, 10 μL of TBS containing 0.2% SDS was added to the washed beads and heated at 95°C for 5 minutes, and the antibodies were inactivated and the MMP-3 was released. The magnetic beads were trapped in one place with a magnet, and the obtained supernatant was recovered.

(1-4) Biotinylated antibody removal process

In the obtained supernatant, not only the MMP-3 but also the inactivated anti-MMP-3 antibodies were coexisted. These antibodies were biotinylated, therefore, can be easily trapped by the SA beads. Accordingly, an eluate, and 10 μL of the SA beads (20 mg/mL) washed three times in advance with a reaction buffer were added to a 1.5 mL volume tube, the resultant mixture was subjected to a biotin-avidin reaction at 4°C for 30 minutes, and the magnetic beads were trapped in one place with a magnet immediately after the reaction. The obtained supernatant was recovered and made into an MMP-3-IP product (20 μL).

(1-5) Results

In order to estimate the recovered amount of each sample, Western blotting (WB) was performed using the above-described biotinylated antibodies. When the lower limit of the detection of the proteins by WB was calculated by using commercially available recombinant human MMP-3 (rMMP-3, manufactured by R&D Systems Inc.) in advance, 50 pg was obtained. Therefore, by loading 50, 100, 200, or 400 pg of rMMP-3 as a standard substance into a gel together with the sample and by performing the WB, semi-quantitative analysis of the amount of the MMP-3 in each sample was performed. In the analysis, 0.1 μL of each sample was loaded into a gel.

As a result, in some of the samples, band intensities were exceeded the band intensity when 400 pg of rMMP-3 was loaded, therefore, each sample was appropriately diluted, and the WB was performed again. The results are shown in FIG. 1.

Band signals of each sample were within the signal range of standard substances, therefore, a calibration curve was created from the signals of the standard substances, and the blood concentration of each sample was calculated in terms of rMMP-3 conversion. Each converted concentration is shown in FIG. 2.

In order to verify the validity of the enrichment, when compared with the MMP-3 level measured in a clinical setting, a strong correlation was shown (right in FIG. 2). This indicates that the MMP-3 level determined through the enrichment process depends on the concentration when the MMP-3 is present in the blood.

In addition, the numerical values calculated this time have a large deviation from the values normally measured in a clinical site, however, this is considered that the standard substance used in the present Examples and the standard substance to be used in the measurement system that are used in a clinical site are different from each other, therefore, the reactivities with antibodies are different from each other.

Further, the same experiment was also performed for the MMP-3 derived from commercially available serum of healthy subjects (provided by Kohjin Bio Co., Ltd.), and the recovery was able to be performed within the MMP-3 level range of healthy subjects reported in a literature.

(Example 2) Glycan profiling by antibody-overlay-lectin array

In the present Example, to the MMP-3 in a serum sample, MMP-3-IP, that had been subjected to simplified fractionation and concentration (enrichment) of MMP-3 in Example 1, the comparative glycan profiling by a lectin array was applied, and the MMP-3 glycan changes associated with rheumatism were examined.

Each MMP-3-IP sample obtained in Example (1-4) was taken in an amount of 2 $\mu$L, and the sample was adjusted to 60 $\mu$L with phosphate-buffered saline (PBSTx) containing 1% Triton X-100 which is a lectin array reaction buffer. The solution was added to each reaction vessel (7 reaction vessels had been formed per glass slide) of a lectin microarray (LecChip (trademark) (manufactured by Glyco Technica Ltd.)), and the resultant mixture was subjected to interaction at 20°C for 10 hours or more. As a result, the binding reaction between the MMP-3 glycan in the sample and the 45 kinds of the lectins immobilized on the array substrate reached the equilibrium state. After that, into the resultant mixture, in order to prevent the glycan of antibodies for detection from binding to unreacted lectins on the substrate and generating noise signals, 2 $\mu$L (equivalent to 20 $\mu$g) of human serum-derived IgG solution (manufactured by Sigma-Aldrich Co. LLC.) was added, and the resultant mixture was reacted for 30 minutes. After performing this blocking reaction, each reaction vessel was washed three times with 60 $\mu$L of PBSTx, and then 2 $\mu$L of human serum-derived IgG solution was added again to 58 $\mu$L of the PBSTx solution. After slightly stirring the resultant mixture, anti-MMP-3 antibodies (biotinylated product) for detection were added in an amount equivalent to 100 ng, and the reaction was performed at 20°C for one hour. After the antigen-antibody reaction, each reaction vessel was washed three times with 60 $\mu$L of PBSTx, and then, into the resultant mixture, a PBSTx solution containing Cy-3 labeled streptavidin (manufactured by GE Healthcare) in an amount equivalent to 200 ng was added, and further the resultant mixture was subjected to a reaction at 20°C for 30 minutes. After the reaction, each reaction vessel was washed three times with 60 $\mu$L of PBSTx, and then array scanning was performed with an array scanner GlycoStation (trademark) (manufactured by Glyco Technica Inc.).

As a result, glycan structure information of MMP-3 was able to be obtained. The glycan structure information of the MMP-3 has not been reported so far. Acquired signals were increased and decreased depending on the MMP-3 level in blood. In FIG. 3, a typical example of the data of 30 cases measured is shown. The lectins showing significant signal intensity in all of the cases were only four kinds of ACG, ABA, Jacalin, and ACA. In addition, ACG is a lectin recognizing $\alpha$2, 6 3 sialic acid, and ABA, Jacalin, ACA are lectins recognizing an O-linked glycan that basically has a core 1 structure or a Tn structure as the skeleton, although the detailed specificities are different from one another.

From the above, the MMP-3 glycan structure, as shown in FIG. 4, is assumed to be an O-linked glycan having a core 1 structure or a Tn structure as the skeleton, and a structural group having a different degree of the sialylation.

Next, the correlation between the lectin signals and the MMP-3 level in blood was examined. As shown in FIG. 5, ACG, ABA, and ACA showed relatively strong correlation, but Jacalin does not show any correlation. This divergence in Jacalin was observed also in a case where the correlation between the O-linked glycan recognition lectins was examined (FIG. 6). It has revealed in analysis of other glycoproteins (Non-Patent Document 14) that each of these three O-linked glycan recognition lectins has a difference in the specificity, and further the tolerance is different and the reactivity of Jacalin decreases most particularly in a case where $\alpha$2,6 sialic acid (NeuNAc) modification is introduced into GalNAc having a core 1 structure. Specifically, in a case of Jacalin, introduction of the modification of $\alpha$2,6 sialic acid (NeuNAc) into GalNAc having a core 1 structure of an O-linked glycan is not acceptable, however, in a case of each of the ABA and ACA, the introduction is acceptable, or is irrelevant.

From the above, it was suggested that the MMP-3 O-linked glycan, with which the blood concentration is increased associated with the affection with rheumatoid arthritis, is rich in the sialylation, particularly $\alpha$2, 6 sialylation to the core 1 structure as compared with that of the MMP-3 also present in serum of healthy subjects.

It is interesting that this phenomenon does not correlate with the MMP-3 level, and after that, it was decided to examine the correlation with the clinical parameters.

(Example 3) Comparative analysis between acquired data and clinical parameters

The correlation between the lectin signals unique to MMP-3 obtained in (Example 2) and the clinical parameters was examined. For verification, among the glycosylation discrimination indexes X that reflect the qualitative changes of the O-linked glycan of proteins, the value in a case of X = ABA / Jacalin was used. Comparison with the parameter showing the activity of rheumatoid arthritis among the verified parameters was shown FIG. 7. Parameters (DAS28-CRP, DAS28-ESR, CDAI, SDAI, and doctor VAS) except for patient VAS showed some degree of correlation. So far, the MMP-3 level has been reported to be weakly correlated with the disease activity, therefore, it is extremely interesting that the ABA/Jacalin values reflecting qualitative changes in the glycan of protein correlate with the disease activity in contrast.

Next, each patient was categorized into four stages (remission, low disease activity, moderate disease activity, and high disease activity) with three evaluation methods (DAS28-ESR, SDAI, and CDAI) representing the disease activity of rheumatoid arthritis, and the correlation between the disease activity and the ABA/Jacalin value was graphed (FIG. 8).

As a result, the ABA/Jacalin value that is a glycosylation discrimination index was correlated well with the disease activity index (the P value obtained with a Spearman rank correlation coefficient is $P < 0.001$). On the other hand, the MMP-3 level was also graphed, but did not show any correlation with disease activities in any of the evaluation methods.

From the above-described results, a possibility that quantitative measurement of the qualitative changes of the MMP-3 O-linked glycan as glycosylation discrimination indexes can be applied to evaluate the disease activity of rheumatoid arthritis has been found for the first time.

In order to verify whether or not the qualitative changes of the glycan are due to the difference in the manners of or changes in the ratios of the sialylation, it was decided to compare and analyze the MMP-3 before and after sialidase digestion with a lectin array.

(Example 4) Influence of sialidase digestion on glycan profile

From the results of (Example 3), it is considered that it is highly possible that changes in the reactivity of MMP-3 in serum to each lectin are generated due to the difference in the manners of or changes in the ratios of the sialylation to MMP-3 O-linked glycan. Accordingly, in the present Example, among the serum samples utilized in the analysis of (Example 1) and (Example 2), 6 cases from each group of a low value group and a high value group of glycosylation discrimination indexes (ABA/Jacalin value) were selected to be 12 cases in total, and influence on the glycan profile was examined in a case where the sialic acid residues of MMP-3 were cleaved and removed in each serum sample.

(4-1) Enrichment of MMP-3 in serum

In accordance with the method in (Example 1), MMP-3 in serum was again enriched in the serum samples from the selected 12 cases, and 12 kinds of the MMP-3-IP samples to be tested were obtained. Names of the samples in the low value group of glycosylationdiscrimination index (ABA/Jacalin value) areRA07, 20, 31, 33, 34, and 35, and names of the samples in high value group of glycosylation discrimination index (ABA/Jacalin value) are RA10, 13, 14, 17, 21, and 24.

(4-2) Sialidase digestion

An MMP-3 IP solution in an amount of 2 $\mu$L from 20 $\mu$L of the obtained MMP-3 IP solution was used for sialidase digestion. Into 2 $\mu$L of the solution, 2 $\mu$L of dedicated 5x reaction buffer B and 0.5$\mu$L (final: 0.125 U/mL) of Sialidase A (both manufactured by Prozyme Inc.) were added, and the resultant mixture was adjusted to 10 $\mu$L with ultrapure water. The reaction was performed at 37°C for one hour, and sialic acid (both $\alpha$2, 6 3 sialic acid and $\alpha$2,6 sialic acid) was cleaved off from MMP-3. As a control, a reaction mixture not containing sialidase among the above compositions was also prepared, and reacted also. After completion of the reaction, all of the reaction mixture was left to stand on ice for a while. The obtained product was used as a sialidase digestion product.

(4-3) Glycan profiling by antibody-overlay-lectin array

An antibody-overlay-lectin array was performed in the similar manner as in Example 1. Inaddition, a sialidase reaction mixture was prepared in advance so as to have a total volume of 60 $\mu$L with PBSTx, and used for lectin array analysis.

The results are shown in Table 9. After the cleavage of sialic acid, the signals became almost zero with a lectin (ACG, or MAH) that recognizes sialic acid of O-linked glycan. Along with that, signals of PNA that is a lectin recognizing a core 1 structure and does not accept any sialylation, appeared in all of the samples. On the other hand, signals were not obtained even after sialic acid digestion in HPA, VVA, and GALI that are lectins recognizing a Tn structure.

From the above, it was understood that the major O-linked glycan structure possessed by MMP-3 is a core 1 structure (Gal ($\beta$1, 3) GalNAc) , and modification of $\alpha$2, 3 sialic acid to Gal is almost accomplished. In addition, it was concluded that the degree of the $\alpha$2, 6 sialylation to GalNAc is less frequency, and the proportion varies along with the pathological conditions. According to this, it is demonstrated that the degree of the $\alpha$2,6 sialylation to GalNAc in a core 1 structure becomes a biomarker of disease activity of rheumatoid arthritis, and the Jacalin-derived signals become a negative activity marker, and further together with the ABA and ACA-derived signals, the ACG-derived signals also show the behavior similar to that of the MMP-3 level in serum. That is, as the "measured signals corresponding to the total amount of MMP-3 in a serum sample" used for the calculation of glycosylation discrimination index X or Y, together with the measurement value of the abundance of MMP-3, the signal level measured by using an anti-MMP-3 antibody, and the measurement signal level showing a binding amount of the MMP-3 and any lectin selected from the group consisting of ABA, ACA, and ACG in a serum sample can be used.

Further, the ABA standardizing glycan profile after sialic acid digestion was almost matched, therefore, it was suggested that the reactivity difference generated is each sample is attributed to the manners of, and the degree of the sialylation(see the structural changes before and after the assumed reaction shown on the right in FIG. 9).

(Example 5) <u>Glycan profiling of MMP-3 in synovial fluid</u>

The MMP-3 produced from a synovial tissue to be a lesion site is released into a synovial fluid, and becomes involved in bone destruction. Part of the MMP-3 moves into blood, and on the premise that the MMP-3 level in serum has a correlation with the production amount in a synovial tissue, it is considered that the MMP-3 level in serum has been conventionally utilized as a biomarker. However, it remains a matter of speculation whether or not the changes in the manners of and the degrees of the sialylation in MMP-3 O-linked glycan in serum, in which a correlation with disease activity of rheumatoid arthritis was found by lectin array analysis in (Example 2), accurately reflect the changes in a structure of glycan of the MMP-3 produced in a synovial tissue.

Accordingly, in the present Example, by examining the glycan profile of the MMP-3 released into a synovial fluid, the validity of the relevance of the glycan changes of MMP-3 in blood to the disease activity of rheumatoid arthritis was tried to be verified.

(5-1) Enrichment of MMP-3 in synovial fluid

In accordance with the method in Example 1, MMP-3 was enriched from a synovial fluid for three cases of rheumatoid arthritis (RA) and three cases of osteoarthritis (OA). In addition, the MMP-3 level in a synovial fluid is largely different between RA and OA, therefore, the MMP-3 level in a synovial fluid was measured in advance, and the enrichment was performed by utilizing the same MMP-3 amount. As a result, as shown in FIG. 10, the obtained MMP-3 levels were almost the same as each other.

(5-2) Glycan profiling by antibody-overlay-lectin array

An antibody-overlay-lectin array was performed in the similar manner as in Example 2. The results are shown in Table 11.

It is extremely interesting that the glycan profiles of RA MMP-3 and OA MMP-3 were significantly different. The difference was able to be explained by four lectins in the similar manner as in the MMP-3 in serum. The MMP-3 derived from a RA patient had relatively weak Jacalin signals and the ACG tended to be strong (FIG. 12). The tendency of the Jacalin signals was consistent with the attenuation of the Jacalin signals associated with the disease activity, which was obtained in serum of a patient with rheumatoid arthritis.

From the above, synovial cells increase the release amount of MMP-3 with the activation, but changes in cellular glycan synthesis machinery due to the activation are generated, as a result of which, it was suggested that changes are generated in the sialylation of O-linked glycan on a protein, in particular in modification of $\alpha$2,6 sialic acid, and becomes high. In addition, it was demonstrated that the findings obtained in the serum samples in Examples 2 and 3 accurately reflect the changes in a glycan structure in MMP-3 produced from a synovial tissue.

(Example 6) Ability to discrimination of MMP-3 positive diseases

(6-1) Comparison of reactivity with MMP-3 O-linked glycan of Jacalin or the like using serums derived frompatients with other rheumatic diseases

In the present Example, for the serum samples from 14 cases of patients with rheumatoid arthritis (RA), from 8 cases of patients with polymyalgia rheumatica (PMR), from 7 cases of patients with relapsing polychondritis (RP), and from 5 cases of healthy control subjects (HC), verification of Examples 1 and 2 was performed. All of the RA, PMR, and RP are diseases that tend to have high MMP-3 level in serum. In clinical diagnosis, RA and PMR often have difficulty in the discrimination from each other, although RP has relatively characteristic clinical symptoms, there is no high sensitivity test method. Specifically, MMP-3 from the serum of a patient with each of the target diseases was concentrated in the same manner as in Example 1, and glycan profiling of the MMP-3 derived from each disease was performed in the similar manner as in Example 2.

As a result, glycan structure information for each disease was able to be obtained. From the data obtained from all of the patients, firstly, when ACG, ABA, ACA, and Jacalin values were compared and examined, as shown in FIG. 13, similarly to the results (FIG. 5) with the serum of a patient with rheumatism targeted in Example 1 (1-1), results that ACG, ABA, and ACA correlate with MMP-3 level, but Jacalin does not correlate with the MMP-3 level were obtained.

In order to observe which disease generates the dissociation between the Jacalin and the MMP-3 level, graphs divided for each disease were created (FIG. 14). As a result, interestingly, the tendency differs between RA and non-RA, in RA, as the MMP-3 level increases, the tendency of the divergence from the Jacalin value increases, and rather it seems that it is decreasing, but on the other hand, such a tendency is not observed in non-RA, and the correlation between the Jacalin and the MMP-3 level was high.

Accordingly, taking the ratio of ACG to Jacalin, wherein ACG is a typical lectin showing a high correlation with MMP-3 level in all of the rheumatic diseases, when ACG/Jacalin values were calculated and graphed (FIG. 15), only the group of having high RA activity showed the high value.

By the way, as demonstrated in Example 4, it is understood that the decrease in Jacalin value reflects the $\alpha 2$, 6 sialylation in GalNAc of O-linked glycan containing MMP-3. In addition, it was able to be demonstrated again also in the present Example that the phenomenon of the decrease in Jacalin value reflecting such $\alpha 2$, 6 sialylation of GalNAc is specific to RA even among the rheumatic diseases, and it is characteristic of a case of highly active RA.

(6-2) Comparison of reactivity with lectin recognizing polylactosamine structure

As in (6-1), it was able to be proved again that the Jacalin signals shows the activity of RA, but it is suggested that the Jacalin signals are also used for the discrimination between the RA and the non-RA, the differences in signals of other lectins were also compared and examined again (FIG. 16).

As other lectins, as a result of lectin array profiling in (6-1), lectins of LEL (derived from *Lycipersicon esculentum*), and STL (derived from *Solanum tuberosum*), both recognize a polylactosamine structure were selected as statistically significant difference was observed between these profiles. Both lectins are considered to recognize GlcNAc residue of a polylactosamine structure.

When the signals of LEL and STL lectin reactive glycans were measured, and determined by a Steel-Dwass test, a result that the signals of PMR and RP were significantly higher than those of HC and RA was obtained, and it was found that there is a large difference for each disease (upper part in FIG. 16) .

The similar Steel-Dwass test was performed for each disease withMMP-3 level in serum, but such a difference cannot be observed only with the MMP-3 level in serum (lower left part in FIG. 16) .

It was analyzed whether or not HC-RA and PMR, and HC-RA-PMR and RP can be discriminated using these signals, according to the ROC curve (receiver operating characteristic curve) plotted by taking the true positive rate on the vertical axis and the false positive rate on the horizontal axis. When ROC analysis was performed for the PMR that is most difficult to be discriminated from RA among the three groups, it was found to have extremely high discrimination ability of ROC = 1.0 and AUC (area under the ROC curve) = 1.0 (lower right part in FIG. 16) . It was found that both signals of STL and LEL have extremely high discrimination ability of AUC = 1.0 with PMR and RA/HC or RP and PMR/RA/HC (the same graph in lower right part in FIG. 16).

That is, it was resulted in that the LEL lectin-reactive MMP-3 glycan, and/or STL lectin-reactive MMP-3 glycan in a serum sample show to become an excellent glycan biomarker for each of the polymyalgia rheumatica (PMR) and the relapsing polychondritis (RP).

This means that by the measurement of the reactivity with a LEL or STL lectin in the MMP-3 concentrated fraction of a serum sample, or by the measurement values in a sandwich assay of an anti-MMP-3 antibody and a LEL or STL lectin, the presence or absence of the affection with polymyalgia rheumatica (PMR) and/or the presence or absence of the affection with relapsing polychondritis (RP) can be determined. In addition, this also means the result showing the following: rheumatoid arthritis and polymyalgia rheumatica which were difficult to be discriminated from each other in the past can be accurately discriminated.

It is revolutionary that polymyalgia rheumatica (PMR) and rheumatoid arthritis (RA) that have difficult cases to be discriminated from each other were able to be accurately discriminated, and further a method for accurately diagnosing relapsing polychondritis (RP) to which an accurate diagnosis method had not been established was able to be provided.

The above experimental data utilizing the glycan profiling data of MMP-3 was analyzed in detail, and a method for specifically detecting only RA among the diseases that tend to have high MMP-3 level was searched.

In RA, signals of a lectin recognizing polylactosamine, such as LEL or STL signals are lower than those in PMR and RP, therefore, RA can be discriminated from PMR and RP, but the LEL or STL signals are also low in HC, therefore, the discrimination from HC cannot be achieved. Therefore, with regard to RA, in order to accentuate the difference with the HC, by taking the ratio to the signals of MMP-3 O-linked glycans of ACG, Jacalin, and the like, indexes (for example, Jacalin/LEL and ACG/LEL) that become high value only in the RA were able to be created (upper part in FIG. 17). Based on these signal levels, when the ability to detect only RA among diseases was examined by ROC analysis, it was shown that there is high discrimination ability of AOC = 0.95 to 0.98 (lower part in FIG. 17).

That is, it was indicated that among the rheumatic diseases similar to one another, in order to accurately discriminate rheumatoid arthritis, the ratio of the reactivity of the LEL or STL lectin in the MMP-3 reactive fraction and the O-linked glycan of Jacalin, ACG, or the like of a serum sample may be measured.

**Claims**

1. A method for providing data for determination of disease activity of rheumatoid arthritis, comprising:

   a process of measuring alteration of MMP-3 O-glycan structure in a body fluid sample.

2. The method according to claim 1, wherein
   the measurement of alteration of MMP-3 O-glycan structure is performed by calculating the following glycosylation discrimination index, X or Y:

   ```
   X = measured signal corresponding to the total amount of
   MMP-3 in a body fluid sample / measured signal corresponding
   to the total amount of Jacalin-binding MMP-3 O-linked glycan
   in said body fluid sample,
   ```

   or

   ```
   Y = measured signal corresponding to the total amount of
   Jacalin-binding MMP-3 O-linked glycan in a body fluid sample
   / measured signal corresponding to the total amount of the MMP-3
   in said body fluid sample,
   ```

   the X and Y are obtained by multiplying the measured signal corresponding to the total amount of MMP-3 in a body fluid sample by the reciprocal of measured signal corresponding to the total amount of Jacalin-binding MMP-3 O-linked glycan in said body fluid sample, and by multiplying the reciprocal of measured signal corresponding to the total amount of MMP-3 in a body fluid sample by the measured signal corresponding to the total amount of Jacalin-binding MMP-3 O-linked glycan in said body fluid sample, respectively.

3. The method according to claim 2, wherein the determination is conducted as follows:

as a value of the glycosylation discrimination index X is higher, disease activity of rheumatoid arthritis is evaluated to be higher, or as a value of the glycosylation discrimination index Y is higher, disease activity of rheumatoid arthritis is evaluated to be lower.

4. The method according to claim 2 or 3, wherein
the measured signal corresponding to the total amount of Jacalin-binding MMP-3 O-linked glycan is a value measured by using a Jacalin lectin.

5. The method according to any one of claims 2 to 4, wherein
the measured signal corresponding to the total amount of MMP-3 is expressed as a measurement value of the abundance of MMP-3 in the body fluid sample, or a measurement signal, wherein an intensity of measurement signal changes depending on the abundance of MMP-3 in the body fluid sample.

6. The method according to claim 5, wherein
the measured signal corresponding to the total amount of MMP-3 expressed as a measurement signal is a signal level measured by using an anti-MMP-3 antibody specifically binds to a polypeptide chain of MMP-3, or a signal level indicating an amount of MMP-3 in the body fluid sample bound to a lectin selected from the group consisting of ABA, ACA, and ACG.

7. A method for determining disease activity of rheumatoid arthritis, comprising the following processes (1) to (5):

(1) measuring signals indicating an amount of MMP-3 in a body fluid sample bound to Jacalin;
(2) measuring signals indicating an abundance of MMP-3 in said body fluid sample, or an amount of the MMP-3 in said body fluid sample bound to a lectin selected from the group consisting of ABA, ACA, and ACG or an amount of the MMP-3 in said body fluid sample bound to an anti-MMP-3 antibody;
(3) calculating a glycosylation discrimination index X by multiplying the reciprocal of the measurement value obtained in the process (1) by the measurement value obtained in the process (2), or a glycosylation discrimination index Y by multiplying the measurement value obtained in the process (1) by the reciprocal of the measurement value obtained in the process (2);
(4) comparing the value (X) or (Y) calculated in the process (3) with a reference value ($X_0$) or ($Y_0$);
(5) determining to have high disease activity of rheumatoid arthritis or to be affected with rheumatoid arthritis, in a case of X being higher than the reference value $X_0$ in the process (4) ($X > X_0$), or
determining to have high disease activity of rheumatoid arthritis or to be affected with rheumatoid arthritis, in a case of Y being lower than the reference value $Y_0$ in the process (4) ($Y < Y_0$).

8. The method according to claim 7, wherein
the reference value $X_0$ or $Y_0$ is a value calculated for body fluid samples from healthy subjects through the processes of (1) to (3).

9. A method for determining condition changes over time of disease activity of rheumatoid arthritis of a subject, comprising the following processes (1) to (5):

(1) measuring signals indicating an amount of MMP-3 in a body fluid sample bound to Jacalin;
(2) measuring signals indicating an abundance of MMP-3 in said body fluid sample, or an amount of the MMP-3 in said body fluid sample bound to a lectin selected from the group consisting of ABA, ACA, and ACG or an amount of MMP-3 in said body fluid sample bound to an anti-MMP-3 antibody;
(3) calculating a glycosylation discrimination index X' by multiplying the reciprocal of the measurement value obtained in the process (1) by the measurement value obtained in the process (2), or a glycosylation discrimination index Y' by multiplying the measurement value obtained in the process (1) by the reciprocal of the measurement value obtained in the process (2);
(4) comparing the value (X') or (Y') calculated in the process (3) with a value ($X'_0$) or ($Y'_0$) calculated for a body fluid sample before the test day from the same subject through the processes of (1) to (3);
(5) determining disease activity of rheumatoid arthritis of the subject to be deteriorated, in a case of X' being a numerical value higher than the value before the test day ($X' > X'_0$) or a case of Y' being a numerical value lower than the value before the test day ($Y' < Y'_0$), and disease activity to be improved in a case of X' being a numerical value lower than the value before the test day ($X' < X'_0$) or a case of Y' being a numerical value higher than the value before the test day ($Y' > Y'_0$), in the comparison process of the process (4).

**10.** The method according to claim 9, wherein
the body fluid sample is a body fluid sample derived from a subject after rheumatoid arthritis treatment, and the body fluid sample before the test day is a body fluid sample derived from the same subject before rheumatoid arthritis treatment, and
in the comparison process of the process (4), the rheumatoid arthritis treatment applied to the subject is evaluated to have a therapeutic effect in a case of X' being lower than that before treatment ($X' < X'_0$) or a case of Y' being higher than that before treatment ($Y' > Y'_0$), and is evaluated not to have a therapeutic effect in a case of X' being higher than that before the treatment or no change ($X' \geq X'_0$) or a case of Y' being lower than that before the treatment or no change ($Y' \leq Y'_0$).

**11.** A glycan epitope to be a biomarker for determination of disease activity of rheumatoid arthritis, wherein
the glycan epitope is an MMP-3 O-linked glycan, and a Jacalin non-binding glycan.

**12.** A glycan epitope to be a biomarker for determination of disease activity of rheumatoid arthritis, wherein
the glycan epitope is an MMP-3 O-linked glycan, and a GalNAc structure of the glycan epitope bound to a Ser/Thr residue in an MMP-3 peptide chain is $\alpha$2,6-sialylated.

**13.** A biomarker for determination of disease activity of rheumatoid arthritis, comprising
the glycan epitope according to claim 11 or 12.

**14.** Use of an MMP-3 O-linked glycan for the method of assessing disease activity of rheumatoid arthritis, wherein
a GalNAc structure of the MMP-3 O-linked glycan bound to a Ser/Thr residue in an MMP-3 peptide chain is $\alpha$2,6-sialylated.

**15.** An MMP-3 O-linked glycan for use in a method of determining disease activity of rheumatoid arthritis, wherein
a GalNAc structure of the MMP-3 O-linked glycan bound to a Ser/Thr residue in an MMP-3 peptide chain is $\alpha$2,6-sialylated.

**16.** A reagent for assessing disease activity of rheumatoid arthritis capable of being applied to a body fluid sample, comprising:

a substance which specifically recognizes the glycan epitope according to claim 11 or 12, or MMP-3 containing a plurality of said glycan epitopes.

**17.** The reagent for assessing disease activity of rheumatoid arthritis according to claim 16, wherein
the substance which specifically recognizes the glycan epitope is a substance selected from the group consisting of a glycan-recognizing antibody, a lectin, a glycan-recognizing peptide, and a glycan-recognizing aptamer.

**18.** A reagent for assessing disease activity of rheumatoid arthritis capable of being applied to a body fluid sample, comprising:

a substance which recognizes a glycan containing $\alpha$GalNAc structure bound to a Ser/Thr residue in an MMP-3 peptide chain, but does not recognize said glycan when $\alpha$GalNAc structure therein is $\alpha$2,6-sialylated.

**19.** The reagent for assessing disease activity of rheumatoid arthritis according to claim 18, wherein
the substance which recognizes a glycan containing $\alpha$GalNAc structure bound to a Ser/Thr residue in an MMP-3 peptide chain, but does not recognize said glycan when $\alpha$GalNAc structure therein is $\alpha$2,6-sialylated is Jacalin.

**20.** A kit for assessing disease activity of rheumatoid arthritis, comprising:

the reagent for assessing disease activity of rheumatoid arthritis according to any one of claims 16 to 19; and further
a substance which specifically binds to MMP-3 in a blood sample in an MMP-3 abundance-dependent manner.

**21.** The kit for assessing disease activity of rheumatoid arthritis according to claim 20, wherein
the substance which specifically binds to MMP-3 in the blood sample in an MMP-3 abundance-dependent manner is a lectin selected from the group consisting of ABA, ACA, and ACG, or an anti-MMP-3 antibody.

22. A glycan epitope to be a biomarker for diagnosis of polymyalgia rheumatica (PMR) and/or relapsing polychondritis (RP), wherein
the glycan is an MMP-3 glycan in a body fluid sample, and having binding activity to a lectin which recognizes a polylactosamine structure.

23. The glycan epitope according to claim 22, wherein
the lectin which recognizes a polylactosamine structure is a LEL lectin or a STL lectin.

24. A biomarker for diagnosis of polymyalgia rheumatica (PMR) and/or relapsing polychondritis (RP), comprising
the glycan epitope according to claim 22 or 23.

25. A reagent for diagnosing polymyalgia rheumatica (PMR) and/or relapsing polychondritis (RP) capable of being applied to a blood sample, comprising:

a substance which specifically recognizes the glycan epitope according to claim 22 or 23, or MMP-3 containing said glycan epitopes.

26. A method for providing data for diagnosis of polymyalgia rheumatica (PMR) and/or relapsing polychondritis (RP), comprising:

a process of measuring the abundance of glycan in a body fluid sample having binding activity against an anti-MMP-3 antibody as well as binding activity to a lectin which recognizes a polylactosamine structure, for a body fluid sample.

27. The method according to claim 26, wherein
the lectin which recognizes a polylactosamine structure is a LEL lectin or a STL lectin.

28. The method according to claim 26 or 27, wherein
the measurement process is performed by a sandwich assay which employs an anti-MMP-3 antibody and a lectin recognizing a polylactosamine structure.

29. A method for determining whether a subject suffers from polymyalgia rheumatica (PMR) and/or relapsing polychondritis (RP), **characterized in that**
a body fluid sample derived from a subject suspected to be suffering from polymyalgia rheumatica (PMR) and/or relapsing polychondritis (RP) is subjected to the following processes (1) to (3):

(1) measuring the abundance of glycan in the body fluid sample having binding activity against an anti-MMP-3 antibody as well as binding activity to a lectin which recognizes a polylactosamine structure;
(2) measuring the abundance of glycan in a body fluid sample derived from a healthy subject and/or a patient with rheumatoid arthritis having binding activity against an anti-MMP-3 antibody as well as binding activity to a lectin which recognizes a polylactosamine structure; and
(3) determining to be suffering from polymyalgia rheumatica (PMR) and/or relapsing polychondritis (RP) in a case where a numerical value showing the abundance of glycan obtained in (1) significantly exceeds a numerical value showing the abundance of glycan obtained in (2).

30. The method according to claim 29, wherein the measurement processes of (1) and (2) are performed by a sandwich assay which employs an anti-MMP-3 antibody and a LEL lectin or STL lectin.

31. A method for providing data for diagnosis of rheumatoid arthritis (RA), comprising:

applying to a body fluid sample at least the following measurement processes (1) and (2):

(1) measuring the abundance of glycan in a body fluid sample having binding activity against an anti-MMP-3 antibody as well as binding activity to a lectin which recognizes a polylactosamine structure; and
(2) measuring the abundance of glycan in said body fluid sample having binding activity against an anti-MMP-3 antibody as well as binding activity to at least one O-linked glycan-recognition lectin selected from the group consisting of Jacalin, ABA, ACA, and ACG.

**32.** The method according to claim 31, wherein
the measurement processes (1) and (2) are performed simultaneously by a sandwich assay which employs said anti-MMP-3 antibody, and a lectin array containing a lectin which recognizes a polylactosamine structure and said O-linked glycan-recognition lectin.

**33.** A method for determining whether a subject suffers from rheumatoid arthritis, **characterized in that**
a body fluid sample derived from a subject suspected to be suffering from rheumatoid arthritis is subjected to the following processes (1) to (4):

> (1) measuring the abundance of glycan in the body fluid sample having binding activity against an anti-MMP-3 antibody as well as binding activity to a lectin which recognizes a polylactosamine structure;
> (2) measuring the abundance of glycan is said body fluid sample having binding activity against an anti-MMP-3 antibody as well as binding activity to at least one O-linked glycan-recognition lectin selected from the group consisting of Jacalin, ABA, ACA, and ACG;
> (3) calculating a ratio of the abundance of glycan obtained in (2) to the abundance of glycan obtained in (1); and
> (4) determining the subj ect to be suffering from rheumatoid arthritis in a case that the numerical value of ratio obtained in the process (3) is significantly higher than a numerical value of ratio of healthy subjects which is obtained by applying the processes (1) to (3) to a body fluid sample derived from a healthy subject in advance or at the same time.

**34.** The method according to claim 33, wherein
the measurement processes (1) and (2) are performed simultaneously by a sandwich assay which employs said anti-MMP-3 antibody, and a lectin array containing a lectin which recognizes a polylactosamine structure and said O-linked glycan-recognition lectin.

**35.** A kit for diagnosis of polymyalgia rheumatica (PMR) and/or relapsing polychondritis (RP), comprising the following (1) and (2) :

> (1) an anti-MMP-3 antibody; and
> (2) a lectin recognizing a polylactosamine structure.

**36.** The kit according to claim 35, wherein
the lectin recognizing a polylactosamine structure is a LEL lectin or a STL lectin.

**37.** A kit for diagnosis of rheumatoid arthritis, comprising the following (1) to (3):

> (1) an anti-MMP-3 antibody;
> (2) a lectin recognizing a polylactosamine structure; and
> (3) at least one lectin recognizing an O-linked glycan selected from the group consisting of Jacalin, ABA, ACA, and ACG.

**38.** The kit according to claim 37, wherein
lectins of (2) and (3) are present in the same lectin array.

[FIG. 1]

[FIG. 2]

| Sample ID | Signal | rMMP3 conversion MMP3 level in serum (ng/mL) |
|---|---|---|
| R07 | 77412 | 558 |
| R08 | 95104 | 650 |
| R09 | 160342 | 3957 |
| R10 | 246722 | 2961 |
| R11 | 224610 | 2446 |
| R12 | 171178 | 602 |
| R13 | 191663 | 1680 |
| R14 | 136664 | 1732 |
| R15 | 216682 | 4091 |
| R16 | 233560 | 1114 |
| R17 | 318072 | 3140 |
| R18 | 319317 | 3153 |
| R19 | 250162 | 301 |
| R20 | 255402 | 616 |
| R21 | 136745 | 654 |
| R22 | 152041 | 884 |
| R23 | 107783 | 637 |
| R24 | 153338 | 3566 |
| R25 | 133258 | 779 |
| R26 | 160121 | 929 |
| R27 | 129453 | 758 |
| R28 | 362796 | 8585 |
| R29 | 230964 | 1351 |
| R30 | 166411 | 873 |
| R31 | 354600 | 2467 |
| R32 | 224665 | 1299 |
| R33 | 212423 | 2377 |
| R34 | 258440 | 1602 |
| R35 | 201218 | 544 |
| R36 | 145445 | 767 |
| Healthy subject serum 1 | 194522 | 514 |
| Healthy subject serum 2 | 147054 | 350 |

$y = 5.4711x + 112.95$

$R^2 = 0.7038$

Conversion value after WB (ng/mL)

Normally measured value (ng/mL)

[FIG. 3]

[FIG. 4]

Disialyl core 1

Sialyl core 1

Core 1

Sialyl Tn

Tn

[FIG. 5]

[FIG. 6]

[FIG. 7]

[FIG. 8]

[FIG. 9]

[FIG. 10]

[FIG. 11]

[FIG. 12]

[FIG. 13]

[FIG. 14]

[FIG. 15]

[FIG. 16]

[FIG. 17]

\* P<0.05
(Steel-Dwass test)

ROC
analysis

## INTERNATIONAL SEARCH REPORT

| International application No. |
| --- |
| PCT/JP2015/080522 |

**A. CLASSIFICATION OF SUBJECT MATTER**
*G01N33/564*(2006.01)i, *C07K14/42*(2006.01)i, *C12N9/64*(2006.01)i, *G01N33/53*(2006.01)i, *G01N33/573*(2006.01)i

According to International Patent Classification (IPC) or to both national classification and IPC

**B. FIELDS SEARCHED**

Minimum documentation searched (classification system followed by classification symbols)
G01N33/564, C07K14/42, C12N9/64, G01N33/53, G01N33/573

Documentation searched other than minimum documentation to the extent that such documents are included in the fields searched
Jitsuyo Shinan Koho            1922–1996   Jitsuyo Shinan Toroku Koho   1996–2016
Kokai Jitsuyo Shinan Koho   1971–2016   Toroku Jitsuyo Shinan Koho   1994–2016

Electronic data base consulted during the international search (name of data base and, where practicable, search terms used)
JSTPlus/JMEDPlus/JST7580(JDreamIII), CAplus/MEDLINE/BIOSIS(STN)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2009/096403 A1  (National University Corporation Hokkaido University), 06 August 2009 (06.08.2009), abstract & US 2010/0297682 A1 abstract & EP 2256499 A1          & EP 2535719 A2 | 1-38 |
| A | JP 2013-44657 A  (Osaka University, 04 March 2013 (04.03.2013), claim 6 (Family: none) | 1-38 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 12 January 2016 (12.01.16) | 26 January 2016 (26.01.16) |

| Name and mailing address of the ISA/ Japan Patent Office 3-4-3,Kasumigaseki,Chiyoda-ku, Tokyo 100-8915,Japan | Authorized officer Telephone No. |
| --- | --- |

Form PCT/ISA/210 (second sheet) (January 2015)

## INTERNATIONAL SEARCH REPORT

International application No.

PCT/JP2015/080522

C (Continuation). DOCUMENTS CONSIDERED TO BE RELEVANT

| Category* | Citation of document, with indication, where appropriate, of the relevant passages | Relevant to claim No. |
|---|---|---|
| A | Michiyo URATA et al., "A Basic Study of Determination Matrix Metalloproteinase-3 and Carbohydrate in Rheumatoid Factor in Serum for Diagnosis of Rheumatoid Arthritis", The Japanese Journal of Clinical Pathology, 2003, vol.51, no.8, pages 745 to 750 | 1-38 |
| A | VAN DEN STEEN P E et al., Matrix remodelling enzymes, the protease cascade and glycosylation, Biochim Biophys Acta, 2001, Vol.1528 No.2/3, Page.61-73, Fig. 4 | 1-38 |
| A | SAARINEN J et al., N-Glycan structures of matrix metalloproteinase-1 derived from human fibroblasts and from HT-1080 fibrosarcoma cells, Eur J Biochem, 1999, Vol.259 No.3, Page.829-840, Abstract | 1-38 |
| A | Shuo T et al., Detection of the heterogeneous O-glycosylation profile of MT1-MMP expressed in cancer cells by a simple MALDI-MS method, PLoS One, 2012, Vol.7 No.8, Page.e43751, Abstract | 1-38 |
| A | JP 10-115618 A (Fuji Chemical Industries, Ltd.), 06 May 1998 (06.05.1998), paragraph [0028] (Family: none) | 1-38 |
| A | JP 2864219 B2 (Fuji Chemical Industries, Ltd.), 03 March 1999 (03.03.1999), paragraph [0024] (Family: none) | 1-38 |
| A | Akira TOGAYACHI et al., "Functional Analysis of β1,3-N-Acetylglucosaminyltransferases and Regulation of Immunological Function by Polylactosamine", Trends Glycoscience Glycotechnology, 2012, vol.24, no.137, pages 95 to 111 | 22-38 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012144512 A
- JP 2014170012 A
- JP 2011157301 A
- JP 2010286279 A
- JP 2007325595 A
- JP 2007082545 A
- JP 2007075106 A
- WO 0252007 A
- JP 2003024099 A
- JP 2003026598 A
- JP 11094836 A

- WO 2008108723 A
- WO 2008001944 A
- WO 9717441 A
- WO 2010001632 A
- JP 2004138489 A
- JP 8228795 A
- WO 2009096403 A
- JP 2012152198 A
- WO 2011047358 A
- WO 2011007797 A

### Non-patent literature cited in the description

- **NAKAGAWA T et al.** *Journal of Biological Chemistry,* 2006, vol. 281 (40), 29797-29806
- *Lab. Invest.,* June 1992, vol. 66 (6), 680-690
- *J. Biol. Chem.,* 25 October 1986, vol. 261 (30), 14245-14255
- *Inflammation and Regeneration,* May 2004, vol. 24 (3), 154-160
- *J. Immunol. Res.,* 2014, vol. 2014, 263625
- *Ann. Rheum. Dis.,* April 2010, vol. 69 (4), 631-637
- *Ann. Rheum. Dis.,* March 2014, vol. 73 (3), 492-509
- *Arthritis Rheum.,* May 2012, vol. 64 (5), 1316-1322
- *Arthritis Rheum.,* March 2011, vol. 63 (3), 573-586
- *J. Rheumatol.,* December 2000, vol. 27 (12), 2761-2768

- *Ann. Rheum. Dis.,* February 2002, vol. 61 (2), 161-166
- *Arthritis Care Res.,* 2012, vol. 64, 1794-1803
- *J. Autoimmun.,* February 2014, vol. 48-49, 76-8
- *Arthritis Rheum.,* 03 October 1990, vol. 3 (19), 1493-1500
- *Mol. Cell. Proteomics,* 2009, vol. 8 (1), 99-108
- *Biochemistry,* 06 November 1990, vol. 29 (44), 10261-10270
- *Nature Methods,* 2005, vol. 2, 851-856
- *Clin. Chem.,* January 2011, vol. 57 (1), 48-56
- **KUNO A et al.** *Molecular & Cellular Proteomics,* 2009, vol. 8 (1), 99-108
- *Methods in Enzymology,* 2010, vol. 479, 185-204
- *Methods in Enzymology,* 2010, vol. 478, 181-195